(19) **Europäisches Patentamt** / **European Patent Office** / **Office européen des brevets**

(11) **EP 4 547 301 B1**

(12) **EUROPEAN PATENT SPECIFICATION**

(45) Date of publication and mention
of the grant of the patent:
**03.12.2025   Bulletin 2025/49**

(21) Application number: **23736015.1**

(22) Date of filing: **25.06.2023**

(51) International Patent Classification (IPC):
*A61M 16/00* (2006.01)      *A61M 16/10* (2006.01)
*A61M 16/16* (2006.01)

(52) Cooperative Patent Classification (CPC):
**A61M 16/109; A61M 16/024; A61M 16/16;**
A61M 16/0066; A61M 16/026; A61M 16/161;
A61M 2016/0027; A61M 2016/0039;
A61M 2205/3334; A61M 2205/3358;
A61M 2205/3368; A61M 2205/3393;
A61M 2205/3653; A61M 2205/505; A61M 2205/52;
(Cont.)

(86) International application number:
**PCT/EP2023/067213**

(87) International publication number:
**WO 2024/002917 (04.01.2024 Gazette 2024/01)**

(54) **CONTROLLING THE EVAPORATION RATE OF A HUMIDIFIER WITH ADAPTIVE POWER CONTROL AND METHOD**

STEUERUNG DER VERDUNSTUNGSRATE EINES BEFEUCHTERS MIT ADAPTIVER LEISTUNGSSTEUERUNG UND VERFAHREN

COMMANDE DU TAUX D'ÉVAPORATION D'UN HUMIDIFICATEUR AVEC COMMANDE DE PUISSANCE ADAPTATIVE ET PROCÉDÉ

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **30.06.2022   US 202263357311 P**

(43) Date of publication of application:
**07.05.2025   Bulletin 2025/19**

(73) Proprietor: **Koninklijke Philips N.V.**
**5656 AG Eindhoven (NL)**

(72) Inventors:
• **BOONSTRA, Bonne Lambert**
**5656 AG Eindhoven (NL)**
• **DUINEVELD, Paulus Cornelis**
**5656 AG Eindhoven (NL)**
• **HETE, Bernard F.**
**5656 AG Eindhoven (NL)**
• **KNEPPER, Michael B**
**5656 AG Eindhoven (NL)**

(74) Representative: **Philips Intellectual Property & Standards**
**High Tech Campus 52**
**5656 AG Eindhoven (NL)**

(56) References cited:
WO-A1-2021/135942      US-A1- 2018 250 490
US-A1- 2019 217 044      US-A1- 2020 338 298

(52) Cooperative Patent Classification (CPC): (Cont.)
A61M 2209/02

**Description**

BACKGROUND

The present embodiments relate generally to humidifiers of a heated pass-over type for use in a sleep or respiratory therapy device and more particularly, to controlling the evaporation rate of a humidifier with constant power control and a method thereof.

A humidifier is an important component in a sleep or respiratory device. It increases the relative humidity of the air entering a patient airway such that it is comfortable for the patient to inhale.

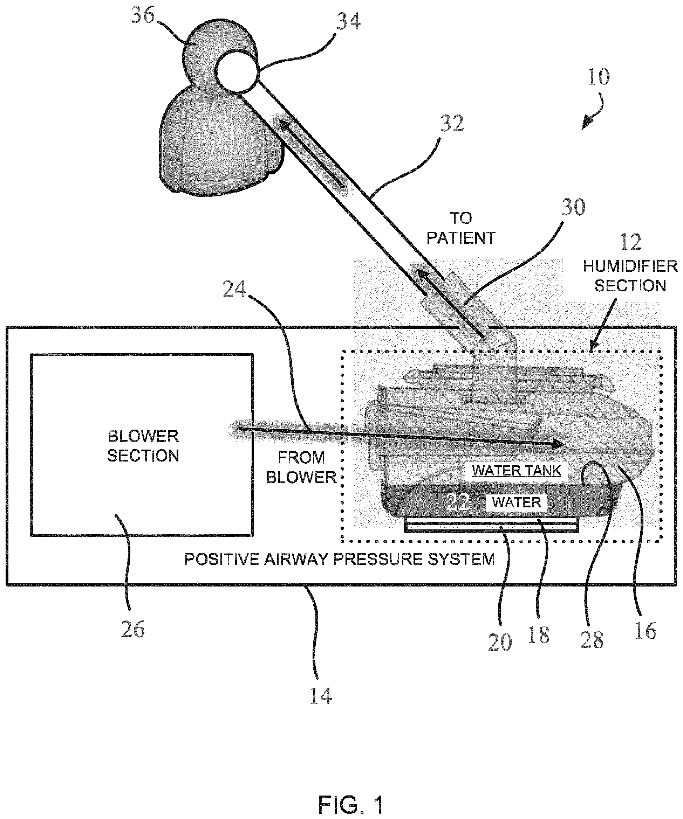

With reference now to **Figure 1,** an example of a current configuration 10 of a pass-over type humidifier 12 for use in a positive airway pressure (PAP) respiratory system 14 consists of a water tank 16 that includes a metal plate 18 in contact with a heater plate 20. The water tank 16 is filled with distilled water 22 by the customer. A flow of breathable air 24 from a blower section 26 enters the water tank 16, where the flow of breathable air 24 is heated up by part of the power dissipation of the fan and motor (not shown) of the blower section 26. The air entering the water tank 16 flows over the water surface 28, picking up heat and moisture. The humidified flow of breathable air 30 leaves the humidifier 12 through a tube or patient circuit 32 that connects to a mask or patient interface 34 worn by a patient 36.

The mass flow of moisture to the patient should be controlled as the required mass flow will depend on the environmental conditions, such as ambient temperature and relative humidity, as well as device settings, such as the average pressure and flow rate generated by the fan, as well as patient parameters. Currently, the evaporation rate to the patient is controlled by setting the heater plate temperature. For each new humidifier type, the evaporation rate is determined completely experimentally for 5 heater plate temperatures (from low to high heater plate temperature) as a function of mask pressure, average air flow rate through the fan, ambient temperature and relative humidity. Hence, there are 5 settings for a customer to select from (i.e., corresponding to heater plate temperature settings from low to high). In the appliance, there is a control algorithm which implements transfer functions (i.e., respectively based upon the completely experimentally determined evaporation rates for the 5 heater plate temperatures) to ensure that the heater plate temperature remains constant at the selected temperature setting during operation.

However, the experiments to determine the respective transfer functions of the control algorithm are tedious as evaporation curves are not simple linear relations and the curves are fully determined experimentally, without using any of the potentially available physical relations. The transfer functions appear to be very sensitive to the contact between the heater plate and the metal plate of the water tank. There appears to be quite some variation in this contact, leading to a varying heat transfer to the water tank, causing a significant variation in evaporation rate.

This variation in evaporation rate was found to be easily $\pm$ 15% or more and also depends on manufacturing tolerances from appliance to appliance. In addition, the variation in evaporation rate can grow over time when calcification or deposition of salts on the metal plate in the water tank will occur. Furthermore, the variation in evaporation rate also requires that several different humidifiers are used for the experimental transfer functions to determine the mean for all expected humidifiers. Moreover, the reported variation in evaporation rate from appliance to appliance remains.

Still further, it appears that the evaporation rate is not constant when the water level in the tank is decreasing, which will result in a varying evaporation rate over time. Finally, the evaporation rate for a given heater plate temperature setting is not constant, as the water mass needs to heat up to reach its steady state signal.

US patent application US 2020/0338298 A1 discloses a humidifier for a respiratory apparatus for delivering a humidified flow of breathable gas to a patient. PCT application WO 2021/135942 A1 discloses a humidification control system and method for a ventilation therapy apparatus. The humidification control system comprises a ventilation therapy apparatus main body, a respiratory humidifier, a heating pipeline and a nasal oxygen cannula. US patent application US 2019/217044 A1 discloses a method of determining a water level in a humidifier chamber that is part of a humidified gases delivery apparatus and system. The method comprising the steps of delivering power to a heater plate, and varying the power delivered to the heater plate. Accordingly, an improved method and apparatus for overcoming the problems in the art is desired.

SUMMARY

According to one aspect, a humidifier of a heated pass-over type for use in a sleep or respiratory therapy device comprises a power control algorithm which is based on the electrical power to the heater plate, instead of the heater plate temperature. By using elementary physics for each humidifier design, a transfer function can be derived where the required power input during steady state can be described as a function of the desired steady state evaporation rate and relevant device and other parameters. The relevant device parameters include heater plate temperature, average pressure generated by the blower/fan, and average flow rate. The other parameters include ambient temperature, relative humidity, and absolute pressure. With the power control algorithm based on the electrical power to the heater plate and the transfer function, it is not only the evaporation rate that can be predicted, but also the steady state water temperature, as well as the temperature of the breathable air entering and exiting the humidifier.

According to the claimed invention, a humidifier of a heated pass-over type for use in a sleep or respiratory therapy device that includes a blower section having a blower for supplying a pressurized flow of breathable gas comprises a heater plate, a water reservoir, one or more sensors, and a controller. The water reservoir is structured to house a volume of water, the water reservoir having a breathable gas inlet and a humidified breathable gas outlet. The water reservoir includes at least one surface for contacting the heater plate. The one or more sensors are configured to generate output signals conveying information about an operating status of the humidifier and ambient conditions.

The controller is configured to control an evaporation rate of the volume of water housed in the water reservoir with a power control according to a power control algorithm for humidifying the flow of breathable gas received at the breathable gas inlet of the water reservoir into a flow of humidified breathable gas at the humidified breathable gas outlet of the water reservoir. The power control algorithm includes a transfer function in which a required power input to the heater plate is a function of a desired evaporation rate based upon generated sensor output signals. It is important that the power, during the unsteady phase, is slightly adjusted based upon the measured sensor outputs until it reaches steady state. During the steady state, there can even be slight adjustments to the required power, when for example, the heater plate temperature slightly changes. In one embodiment, the generated sensor output signals are selected from a device parameter group consisting of heater plate temperature, a pressure generated by the blower, and a flow rate generated by the blower, and an ambient parameters group consisting of ambient temperature, ambient relative humidity, and ambient pressure.

According to an embodiment, the humidifier includes a transfer function which is specific to a given humidifier design and where the required power input to the heater plate during a quasi-steady state can be described as a function of (i) a desired quasi-steady state evaporation rate and (ii) generated sensor output signals. In one embodiment, the generated sensor output signals include: (i) device parameters selected from the group consisting of: a heater plate temperature, a pressure of the breathable gas generated by the blower, and a flow rate of the breathable gas, and (ii) additional parameters of ambient temperature, ambient relative humidity, and ambient pressure.

In another embodiment, the electrical input power, represented as a variable Pow, is determined, via the power control algorithm, as a function of the desired evaporation rate given by a linearized transfer function:

$$Pow = c_0 + c_1 \cdot \dot{m}_w + c_2 \cdot Q + c_3 \cdot \Delta p + c_4 \cdot T_{atm} + c_5 \cdot p_{atm} + c_6 \cdot RH_{atm} + c_7 \cdot T_{hp},$$

wherein

$\dot{m}_w$ is evaporation rate in units of mg/s;
$Q$ is flow rate in units of L/min at standard conditions;
$\Delta p$ is pressure by the blower in units of cmH$_2$O;
$T_{atm}$ is ambient temperature in units of °C;
$p_{atm}$ is ambient pressure in units of mbar;
$RH_{atm}$ is relative humidity in units of %;
$T_{hp}$ is heater plate temperature in units of °C; and
$c_0$, $c_1$, $c_2$, $c_3$, $c_4$, $c_5$, $c_6$, and $c_7$ are predetermined coefficients.

In a further embodiment, the humidifier includes wherein values of water temperature of the volume of water housed in the water reservoir, inlet humidifier temperature of the flow of breathable gas at the breathable gas inlet, and outlet humidifier temperature at the humidified breathable gas outlet are each determined using a respective linearized transfer function:

$$y_{pred} = c_0 + c_1 \cdot Q + c_2 \cdot \Delta p + c_3 \cdot Pow + c_4 \cdot T_{atm} + c_5 \cdot p_{atm} + c_6 \cdot RH_{atm} + c_7 \cdot T_{hp}.$$

According to another embodiment, the water reservoir includes a plate disposed on or integral with the at least one surface of the water reservoir and configured for being placed in contact with the heater plate. In another embodiment, the humidifier includes wherein the required electrical power input to the heater plate comprises an electrical power controlled by pulse width modulation. In a still further embodiment, the humidifier includes wherein the required power input comprises an electric power input to the heater plate that is operable as a control parameter to regulate the evaporation rate. In yet another embodiment, the evaporation rate is continuously adapted to changes in the operating status of the humidifier and the ambient conditions.

According to another embodiment, the humidifier further comprises a shunt resistance in series with the heater plate, wherein the controller is further configured to obtain a measurement of electrical power delivered to the heater plate via measuring a voltage drop across the shunt resistance, determining electrical current to the heater plate using the measured voltage drop across the shunt resistance and voltage, $V_{hp}$, across the heater plate.

According to the claimed invention, the power control algorithm includes an accelerated steady state function for use in obtaining a steady state equilibrium faster than without the accelerated steady state function, wherein the controller, via the power control algorithm, is configured to (i) initially set a power input to the heater plate to an increased power input compared to a quasi-steady state, and (ii) responsive to a detection of at least one of the generated sensor output signals attaining at least a predetermined percentage of its quasi-steady state value, switch to quasi-steady state power control algorithm power level corresponding to quasi-steady state settings, wherein the controller is configured to apply the increased power input for a fixed time, wait an amount of time as a temperature of the heater plate becomes equal to a temperature of the water, and estimate a timing for the increased power input from a difference between the temperature of the heater plate and an expected temperature of the steady state equilibrium.

In yet another embodiment, the humidifier includes wherein the controller is further configured, responsive to a detection of an increase in heater plate temperature above a threshold amount, to reduce the power input to the heater plate.

According to one embodiment, a gas delivery system for delivering a pressurized flow of humidified breathable gas to a patient via a patient circuit, comprises a blower assembly and a humidifier. The blower assembly includes a blower adapted to generate the pressurized flow of breathable gas, and a gas flow path including an inlet and an outlet. The humidifier comprises an embodiment or combination of embodiments as described herein. There is provided a fluidic coupling between the blower, the humidifier, and the patient circuit.

According to yet another embodiment, a method of humidifying a flow of breathable gas in a sleep or respiratory therapy device utilizes a humidifier of a heated pass-over type. The sleep or respiratory therapy device includes a blower section having a blower for supplying a pressurized flow of breathable gas. The method comprises providing a heater plate and a water reservoir. The water reservoir is structured to house a volume of water, the water reservoir having a breathable gas inlet and a humidified breathable gas outlet. The water reservoir includes at least one surface configured for being in contact with the heater plate. The method further includes providing one or more sensors configured to generate output signals conveying information that indicates one or more device parameters selected from the group consisting of heater plate temperature, pressure of the breathable gas, and flow rate of the breathable gas, and other parameters that comprise an ambient temperature, a relative humidity, and ambient pressure.

The method further comprises controlling, via a controller, an evaporation rate of the volume of water housed in the water reservoir with a power control according to a power control algorithm for humidifying the flow of breathable gas received at the breathable gas inlet of the water reservoir into a flow of humidified breathable gas at the humidified breathable gas outlet of the water reservoir, wherein the power control algorithm includes a transfer function in which a required power input to the heater plate is a function of a desired evaporation rate based upon generated sensor output signals. The power control algorithm includes an accelerated steady state function for use in obtaining a steady state equilibrium faster than without the accelerated steady state function. The method further comprises: initially setting, via the power control algorithm a power input to the heater plate to an increased power input compared to a quasi-steady state, and responsive to a detection of at least one of the generated sensor output signals attaining at least a predetermined percentage of its quasi-steady state value, switching to quasi-steady state power control algorithm power level corresponding to quasi-steady state settings, applying the increased power input for a fixed time, waiting an amount of time as a temperature of the heater plate becomes equal to a temperature of the water, estimating a timing for the increased power input from a difference between the temperature of the heater plate and an expected temperature of the steady state equilibrium.

In one embodiment, the method includes wherein the generated sensor output signals are selected from a device parameter group consisting of heater plate temperature, a pressure generated by the blower, and a flow rate generated by the blower, and an ambient parameters group consisting of ambient temperature, ambient relative humidity, and ambient pressure. In another embodiment, the transfer function is specific to a given humidifier design and where the required power input to the heater plate during a quasi-steady state can be described as a function of (i) a desired steady state evaporation rate and (ii) generated sensor output signals.

In yet another embodiment, the method includes wherein the electrical input power, represented as a variable Pow, is determined, via the power control algorithm, as a function of the desired evaporation rate given by a linearized transfer function:

$$Pow = c_0 + c_1 \cdot \dot{m}_w + c_2 \cdot Q + c_3 \cdot \Delta p + c_4 \cdot T_{atm} + c_5 \cdot p_{atm} + c_6 \cdot RH_{atm} + c_7 \cdot T_{hp},$$

wherein

$\dot{m}_w$ is evaporation rate in units of mg/s;
$Q$ is flow rate in units of L/min at standard conditions;

$\Delta\boldsymbol{p}$ is pressure by the blower/fan in units of cmH$_2$O;

$\boldsymbol{T_{atm}}$ is ambient temperature in units of °C;

$\boldsymbol{p_{atm}}$ is ambient pressure in units of mbar;

$\boldsymbol{RH_{atm}}$ is relative humidity in units of %;

$\boldsymbol{T_{hp}}$ is heater plate temperature in units of °C; and

$c_0$, $c_1$, $c_2$, $c_3$, $c_4$, $c_5$, $c_6$, and $c_7$ are predetermined coefficients.

In a further embodiment, values of water temperature of the volume of water housed in the water reservoir, inlet humidifier temperature of the flow of breathable gas at the breathable gas input, and outlet humidifier temperature at the humidified breathable gas outlet are determined each using a respective linearized transfer function:

$$y_{pred} = c_0 + c_1 \cdot Q + c_2 \cdot \Delta p + c_3 \cdot Pow + c_4 \cdot T_{atm} + c_5 \cdot p_{atm} + c_6 \cdot RH_{atm} + c_7 \cdot T_{hp}.$$

In another embodiment, the method includes wherein the water reservoir further has a heat conductive plate disposed on or integral with the at least one surface of the water reservoir and configured for being placed in contact with the heater plate.

The power control algorithm according to the embodiments of the present disclosure possesses a number of advantages over the humidifier control algorithm/method known in the art. One advantage is that the evaporation rate determination is very accurate, as it is not sensitive to the contact between the heater plate and the metal plate in the water tank, or calcification or deposition of salts on the metal plate over time. Another advantage is that the evaporation rate can be continuously adapted (i.e., in contrast to only 5 settings), including an adaptation to different environmental conditions. A further advantage is that the evaporation rate is independent of the water volume in the water tank. Still another advantage is that the power control algorithm, according to one embodiment disclosed herein, offers a faster time to reach steady state equilibrium.

The power control algorithm, according to the embodiments disclosed herein, makes it possible to predict the relative humidity of the breathable air entering a patient mask (or patient interface when a heated tube/patient circuit is used) with a high accuracy.

Still further advantages and benefits will become apparent to those of ordinary skill in the art upon reading and understanding the following detailed description.

BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.

Figure 1 is a simplified block diagram view of a positive airway pressure system including a humidifier of a heated pass-over type for delivering humidified breathing gas to a user;

Figure 2 is a block diagram view of a positive airway pressure system including a humidifier of a heated pass-over type for delivering humidified breathing gas to a user according to an embodiment of the present disclosure;

Figure 3 is a simplified overview diagram of heat and mass transfer in a humidifier according to an embodiment of the present disclosure;

Figure 4 is a table of system parameters of a humidifier according to an embodiment of the present disclosure;

Figure 5 is a table of physical constants in connection with a humidifier according to an embodiment of the present disclosure;

Figure 6 is a table of variables in the system of a humidifier according to an embodiment of the present disclosure;

Figure 7 is a block diagram schematic view of a blower section and humidifier according to an embodiment of the present disclosure;

Figure 8 is a block diagram schematic view of an experimental set-up for a humidifier according to an embodiment of the present disclosure;

Figure 9 is plot of recorded parameters in a detailed example of a measurement run at one setting of a DOE;

Figure 10 is a table of results of a first DOE (DOE$_1$) with variation of flow rate setting $(Q)$, pressure setting $(\Delta p)$, and heater plate power setting $(P_{el})$;

Figure 11 is a table of system parameters determined from the first DOE (DOE$_1$);

Figure 12 is a table of experimental results of a second DOE (DOE$_2$) where ambient conditions have been varied;

Figure 13 is a table of experimental results of a third DOE (DOE$_3$) where ambient conditions and the thermal resistance of the heater plate have been varied;

Figure 14 is a pictorial view of an experimental set-up with an automated servo lung (ASL) mimicking a patient for causing a varying flow rate through the humidifier according to an embodiment of the present disclosure;

Figure 15 is a table of experimental results showing that the evaporation rate and inlet and outlet humidifier temperatures remain constant with varying water level according to an embodiment of the present disclosure;

Figure 16 is a table of variables used in a variation scheme of seven input parameters to determine the linear transfer function according to an embodiment of the present disclosure;

Figures 17-20 are plots of linearization errors for humidifier inlet temperature, humidifier outlet temperature, water temperature, and evaporation rate, respectively, according to an embodiment of the present disclosure;

Figures 21-24 are plots illustrating a comparison between all measurements and predictions of evaporation rate, humidifier outlet temperature, humidifier input temperature, and water temperature, respectively, based on the simplified linear transfer function according to an embodiment of the present disclosure; and

Figure 25 is a plot illustrating an example of power control with a maximum power according to an embodiment of the present disclosure.

## DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

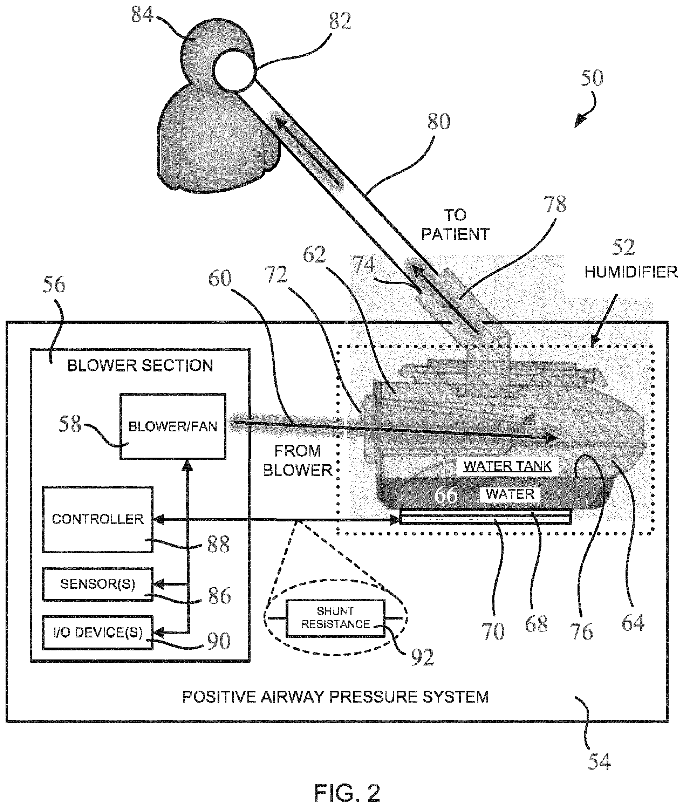

Turning now to **Figure 2,** there is shown a configuration 50 of a pass-over type humidifier 52 of a heated pass-over type for use in a sleep and respiratory therapy device or positive airway pressure (PAP) respiratory system 54 according to an embodiment of the present disclosure. The configuration comprises a blower section 56 having a blower 58 for supplying a pressurized flow of breathable gas 60. The humidifier 52 comprises a water reservoir 62 having a water tank 64 structured to house a volume of water 66. The water reservoir 62 further comprises a metal plate 68 on at least one surface of the water reservoir and/or water tank and is configured for contacting with a heater plate 70. The heater plate 70 can also be configured for receiving power from a power source to extend the embodiments to other heating methods/sources such as induction heating. According to another embodiment, the heater plate can comprise (i) an electric heater plate (i.e., thermal heating method), (ii) an inductive heating coil (i.e., inductive heating method), or combination thereof. The water reservoir 62 further includes a breathable gas inlet 72 and a humidified breathable gas outlet 74.

In one embodiment, the water tank 64 comprises a removable water tank which can be removed from the water reservoir 62, via a suitable opening and closing mechanism (not shown) and filled with a volume of distilled water by the user. The filled water tank 64 is then re-inserted into the water reservoir 62. A pressurized flow of breathable gas 60 from the blower section 56 is input to the humidifier 52, via the breathable gas inlet 72, where the flow of breathable gas 60 is heated up by part of the power dissipation of the fan and blower motor 58 of the blower section 56. The air entering the water reservoir 62 flows over the water surface 76, picking up heat and moisture. The humidified flow of breathable air 78 leaves the humidifier 52 through humidified breathable gas outlet 74 and a tube or patient circuit 80 that connects to a mask or patient interface 82 worn by a patient 84.

Humidifier 52 further comprises one or more sensors 86 configured to generate output signals conveying information that indicates one or more device parameters selected from the group consisting of heater plate temperature, an average pressure of the breathable gas, and an average flow rate of the breathable gas, and other parameters that comprise an ambient temperature, a relative humidity, and ambient pressure. In addition, a controller 88 is configured to control an evaporation rate of the volume of water 66 housed in the water reservoir 62 with an electrical power input control according to a power control algorithm for humidifying the flow of breathable gas 60 received at the breathable gas inlet 72 of the water reservoir 62 into a flow of humidified breathable gas 78 at the humidified breathable gas outlet 74 of the water reservoir 62.

In one embodiment, controller 88 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given humidifier device implementation and/or application. Controller 88 can further comprise one or more various modules configured to implement a given functionality.

According to one embodiment, the controller 88 is configured to control an evaporation rate of the volume of water housed in the water reservoir with a power control according to a power control algorithm for humidifying the flow of breathable gas received at the breathable gas inlet of the water reservoir into a flow of humidified breathable gas at the humidified breathable gas outlet of the water reservoir. The power control algorithm includes a transfer function in which a required power input to the heater plate is a function of a desired evaporation rate based upon generated sensor output signals.

In one embodiment, the controller implements a power control algorithm that includes a linearized transfer function in which a required power input to the heater plate 70 is a function of a desired evaporation rate based upon predetermined device parameters. The linearized transfer function is specific to the blower 58 supplying the flow of breathable gas 60 to the breathable gas inlet 72 of the water reservoir 62, as discussed further herein. In one embodiment, the required electrical power input to the heater plate 70 comprises an electrical power, PWM% in units of %, controlled by pulse width modulation (PWM). In another embodiment, the required power input comprises an electric power input to the heater plate that is operable as a control parameter to regulate the evaporation rate, as will be discussed further herein. In other words, according to the embodiments of the present disclosure, power to the heater plate is actively used to control the evaporation rate. The evaporation rate can further be based on one or more of the operating pressure and blower motor type.

With reference still to **Figure 2,** there is shown one or more input/output device(s), collectively indicated by reference numeral 90, which is representative of any number of suitable I/O devices for inputting or outputting one or more of an audible, visual, and/or tactile input or output, according to the requirements of a given humidifier implementation, further as discussed herein. For example, input/output device(s) 90 can comprise one or more of an input/output device, user interface, tactile output device, touch screen, optical display, microphone, keypad, keyboard, pointing device, image capture device, video camera, audio output device, and any combination thereof, according to the requirements of a given humidifier system implementation.

As illustrated in **Figure 2,** controller 88, sensor(s) 86 and I/O devices 90 are illustrated as being located within the blower section 56. However, the controller 88, sensor(s) 86 and I/O devices 90 can also be located within the humidifier section 52. Still further, the controller 88, sensor(s) 86 and I/O devices 90 can be distributed between both the blower section 56 and the humidifier section 52, according to the requirements of a given humidifier, blower, and/or positive airway pressure system implementation.

In one embodiment, the electrical power to the heater plate 70 can be measured by using a shunt resistance in case there is too much variation of the electrical resistance of the heater plate (i.e., due to different heater plate manufacturers, manufacturing processes and/or tolerances). In particular, a shunt resistance 92 can be placed in series with power to the heater plate 70. The controller 88 is configured to obtain a measurement of electrical power delivered to the heater plate via measuring a voltage drop across the shunt resistance, determining electrical current to the heater plate using the measured voltage drop across the shunt resistance and voltage, $V_{hp}$, across the heater plate.

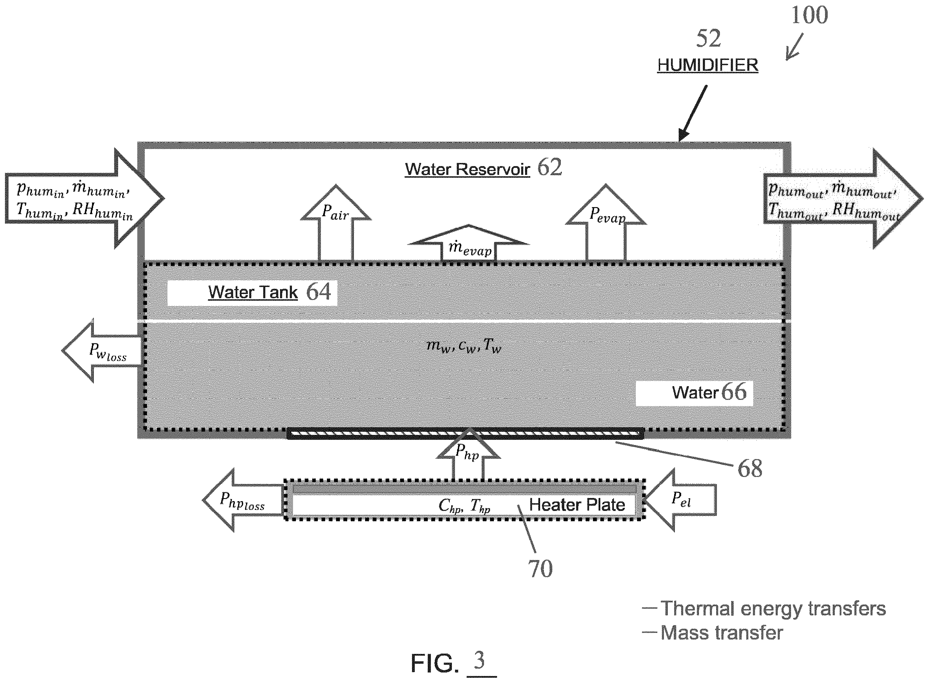

**Figure 3** is a simplified overview diagram 100 of heat and mass transfer in a humidifier 52 according to an embodiment of the present disclosure. As will be discussed further herein, the power control algorithm for implementation via the controller 88 is based on elementary physics of heat and mass transfer as will be discussed. In **Figure 3,** located immediately below the water tank 64 is positioned a heater plate 70, with a certain (low) mass where there is electrical power input ($P_{el}$), thermal losses ($P_{hp\,loss}$) to the environment and power ($P_{hp}$) transmitted to the water reservoir or tank. In that water reservoir, there are thermal losses to the environment ($P_{w\,loss}$) and part of the heat is transmitted to the air ($P_{air}$) flowing over the water. Finally, there is a certain part of the energy used to evaporate water ($P_{evap}$). The heat and mass transfer from the water is picked up by the air ($\dot{m}_{evap}$), changing the temperature and relative humidity of the air flowing over the water.

This is reflected in two coupled differential equations for the heater plate and the water temperature, given by:

$$C_{hp} \cdot \frac{dT_{hp}}{dt} = P_{el} - P_{hp} - P_{hp_{loss}}$$

(Eq. 1)

$$m_w \cdot c_w \cdot \frac{dT_w}{dt} = P_{hp} - P_{air} - P_{evap} - P_{w_{loss}}$$

$$\text{(Eq. 2)}$$

where $C_{hp}$ is the thermal capacity of the heater plate, $m_w$ the water mass and $c_w$ the specific heat of water. Note that for simplicity, we take into account that the change of the water mass is small compared to the change in water temperature. Now the power from the heater plate to the water is given by:

$$P_{hp} = \frac{T_{hp} - T_w}{R_{th}(T_{hp}, T_w, A_{hp}, R_c, \ldots)}$$

$$\text{(Eq. 3)}$$

Where $R_{th}$ is the thermal resistance of the heater plate, which is a steep function of the exact thermal contact between the heater plate and the metal plate in the water tank. We have also found experimentally that this resistance can vary significantly, causing a large variation of the power supply to the water. From equation 2, we learn that in steady state, the power transferred from the heater plate to the water and hence the evaporation rate will vary with varying thermal resistance when the heater plate temperature is controlled, as is the case in the current control strategy.

We can write equations 1 and 2 in a generic form based on heat and mass transfer theory, as follows:

$$C_{hp} \cdot \frac{dT_{hp}}{dt} = \frac{V_{hp}^2}{R_{hp}} \cdot \frac{PWM\%}{100\%} - \frac{T_{hp} - T_w}{R_{th}(T_{hp}, T_w, A_{hp}, R_c, \ldots)} - \alpha_{hp_{loss}} \cdot (T_{hp} - T_{atm})$$

$$\text{(Eq. 4)}$$

$$m_w \cdot c_w \cdot \frac{dT_w}{dt} =$$

$$\frac{T_{hp} - T_w}{R_{th}(T_{hp}, T_w, A_{hp}, R_c, \ldots)} - \alpha_{w_{loss}} \cdot (T_w - T_{atm}) - h(Q, m_w) \cdot A_s(m_w) \cdot (T_w - T_{hum_{in}}) - \dot{m}_w \cdot L_h$$

$$\text{(Eq. 5)}$$

The mass transfer of water will change the air properties, which can be written as:

$$\dot{m}_w = k(Q, m_w) \cdot A_s(m_w) \cdot M_w \cdot \left( \frac{p_{v,s}(T_w)}{R_u \cdot T_w} - \frac{RH_{atm} \cdot p_{v,s}(T_{atm})}{R_u \cdot T_{atm}} \right)$$

$$\text{(Eq. 6)}$$

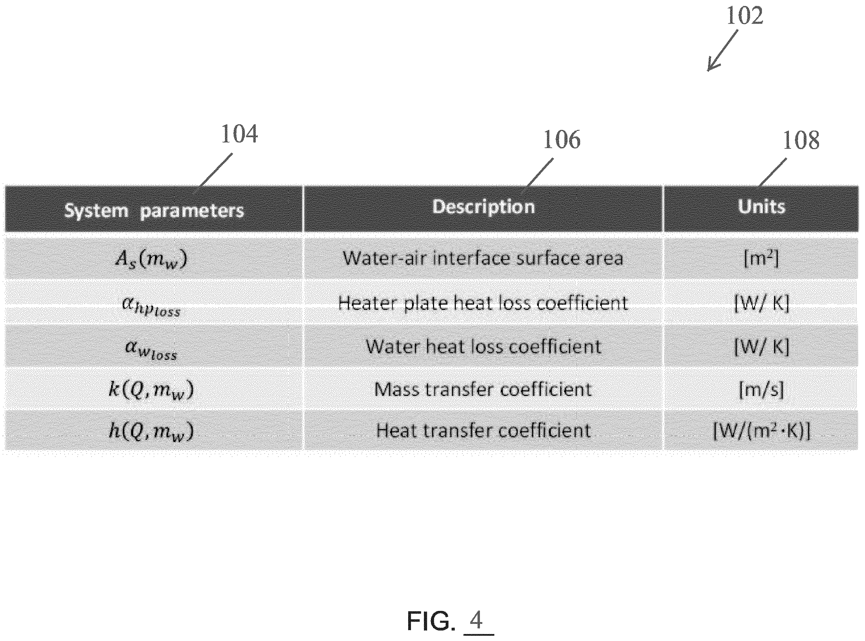

In these three equations (i.e., Eq. 4, 5 and 6), there are quite some parameters, which will be explained. First there are the system parameters, i.e., these are parameters that depend on the humidifier design that will be used. Referring now to Figure 4, a table 102 of the system parameters 104, descriptions 106, and units 108 for a humidifier according to an embodiment of the present disclosure is shown. In particular, the system parameters 104 include: water-air interface surface area ($A_s(m_w)$) in units of [m$^2$]; heater plate heat loss coefficient ($\alpha_{hp\,loss}$) in units of [W/K]; water heat loss coefficient ($\alpha_{w\,loss}$) in units of [W/K]; mass transfer coefficient $k(Q, m_w)$ in units of [m/s]; and heat transfer coefficient $h(Q, m_w)$ in units of [W/(m$^2 \cdot$K)].

Based on the physics, we expect that the heater plate and water heat loss coefficients will be constants. Note that in the water heat loss coefficient, we have also included the effect of other losses, such as the losses from the top of the humidifier to the environment and which are for simplicity not incorporated in the heat and mass transfer model. The heat and mass transfer coefficient from the water to the air will depend in principle on the flow rate $Q$ and water mass $m_w$, however the influence of the water mass appears to be negligible, when using a constant power control method according to the embodiments of the present disclosure.

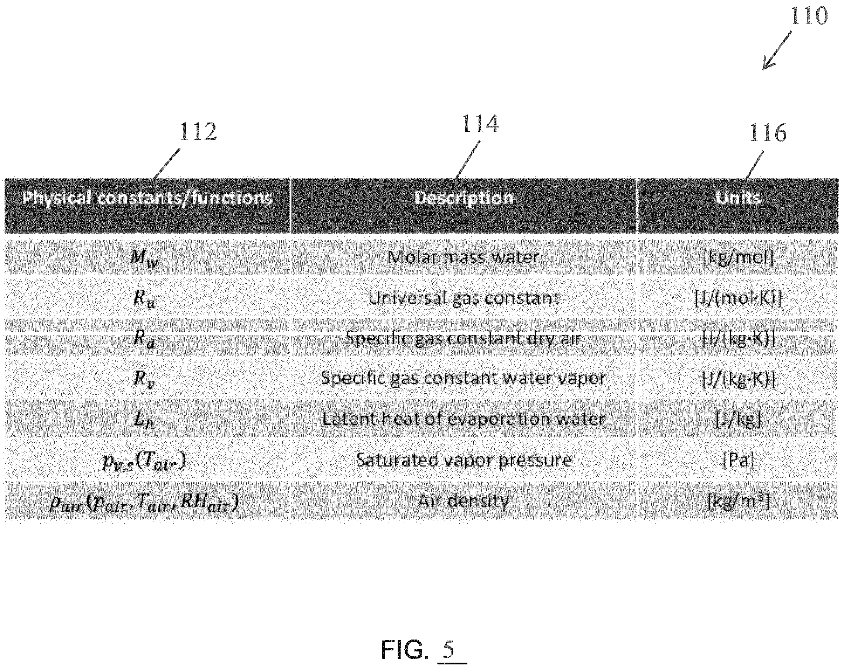

Referring now to **Figure 5.** a table 110 of physical constants/functions 112, descriptions 114, and units 116 is shown in connection with a humidifier according to an embodiment of the present disclosure. These are all physical constants, i.e., parameters that will <u>not</u> vary. In particular, the physical constants include molar mass of water ($M_w$) in units of [kg/mol]; universal gas constant $(\overline{R_u})$ in units of [J/(mol·K)]; specific gas constant of dry air ($R_d$) in units of [J/(kg·K)]; specific gas constant of water vapor ($R_v$) in units of [J/(kg·K)]; latent heat of evaporation water ($L_h$) in units of [J/kg]; saturated vapor pressure ($p_{v,s}(T_{air})$) in units of [Pa]; and air density ($\rho_{air}(p_{air}, T_{air}, RH_{air})$) in units of [kg/m³].

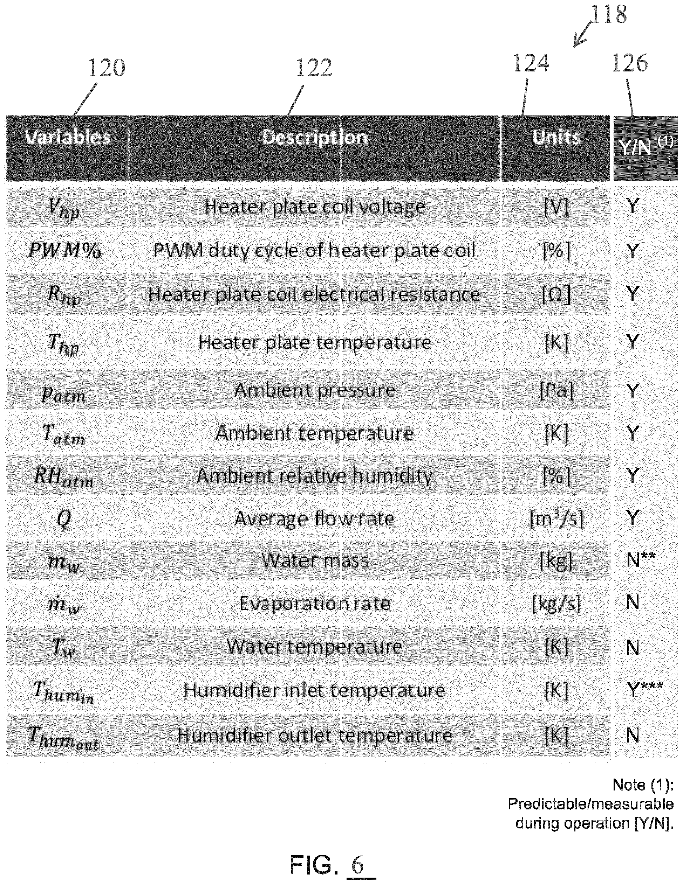

**Figure 6** is a table 118 of all variables 120 in the system, descriptions 122, and units 124 relating to a humidifier according to an embodiment of the present disclosure. In addition, the table indicates, in the last column 126 thereof, which variables are measurable, predictable, or not relevant (indicated by Y), and which are the variables (indicated by N) to be predicted with the model according to the embodiments of the present disclosure. The variables include: heater plate coil voltage ($V_{hp}$) in units of [V]; PWM duty cycle of heater plate coil *(PWM%)* in units of [%]; heater plate coil electrical resistance ($R_{hp}$) in units of [Q]; heater plate temperature ($T_{hp}$) in units of [K]; ambient pressure ($p_{arm}$) in units of [Pa]; ambient temperature ($T_{atm}$) in units of [K]; ambient relative humidity ($RH_{arm}$) in units of [%]; average flow rate (Q) in units of [m³/s]; water mass ($m_w$) in units of [kg]; evaporation rate ($\dot{m}_w$) in units of [kg/s]; water temperature ($T_w$) in units of [K]; humidifier inlet temperature ($T_{hum\,in}$) in units of [K]; and humidifier outlet temperature ($T_{hum\,out}$) in units of [K].

Note that the thermal resistance of the heater plate $R_{th}$ is lacking from the listing of variables in the system. This parameter is eliminated by inserting (Eq. 4) into (Eq. 5) and taking the steady state situation. The result is a system of three non-linear equations, where (Eq. 9) follows from an energy balance of the air flow, i.e., the power transferred to the air flow is used to increase the air temperature.

$$\frac{V_{hp}^2}{R_{hp}} \cdot \frac{PWM\%}{100\%} = \alpha_{hp_{loss}} \cdot \left(T_{hp} - T_{atm}\right) + \alpha_{w_{loss}} \cdot \left(T_w - T_{atm}\right) + h(Q, m_w) \cdot A_s(m_w) \cdot \left(T_w - T_{hum_{in}}\right) + \dot{m}_w \cdot L_h$$

$$(\text{Eq. 7})$$

$$\dot{m}_w = k(Q, m_w) \cdot A_s(m_w) \cdot M_w \cdot \left(\frac{p_{v,s}(T_w)}{R_u \cdot T_w} - \frac{RH_{atm} \cdot p_{v,s}(T_{atm})}{R_u \cdot T_{atm}}\right)$$

$$(\text{Eq. 8})$$

$$\rho_{air_{atm}}(p_{atm}, T_{atm}, RH_{atm}) \cdot Q \cdot c_p \cdot \left(T_{hum_{out}} - T_{hum_{in}}\right) = h(Q, m_w) \cdot A_s(m_w) \cdot \left(T_w - T_{hum_{in}}\right)$$

$$(\text{Eq. 9})$$

Now it turns out from experiments, that will be discussed later, that both the evaporation rate as well as the outlet humidifier temperature are independent of the water mass. Hence, we simplify the system of three equations by taking a constant $A_s$, i.e., contact area of water with the air flow and wherein the variables h and $k$ are only functions of the average air flow rate Q.

In principle, this system of three (3) equations with three (3) unknowns (i.e., water temperature, evaporation rate and outlet humidifier temperature) can be solved. However, before we can do this, we need to know the inlet air temperature in the humidifier (note that this is not equal to the ambient temperature as the air is heated up by the power dissipation of the fan or blower), as well as the five (5) system parameters described in **Figure 4.**

From elementary physics we can show:

$$T_{hum_{in}} = T_{atm} + \frac{f(\Delta p, Q)}{\rho_{air_{atm}}(p_{atm}, T_{atm}, RH_{atm}) \cdot Q \cdot c_p}$$

$$(\text{Eq. 10})$$

Here $f(\Delta p, Q)$ is the power transferred from the fan to the air passing through the fan, which is a function of the average pressure and the average flow rate generated by the fan. The parameter $f(\Delta p, Q)$ is a system parameter that only depends on the air path construction and the fan type being used.

The six (6) system parameters can be determined experimentally via a DOE (Design of Experiments). There are only three (3) important system parameters that can vary, which are the *average* fan pressure $\Delta p$, the average flow rate $Q$, and the electrical power to the heater plate $P_{el}$. This requires preferably at least eight (8) experiments where these three (3) parameters are varied over the complete expected range. During these experiments, all parameters are logged, including the water temperature, evaporation rate, humidifier inlet air temperature and humidifier outlet air temperature.

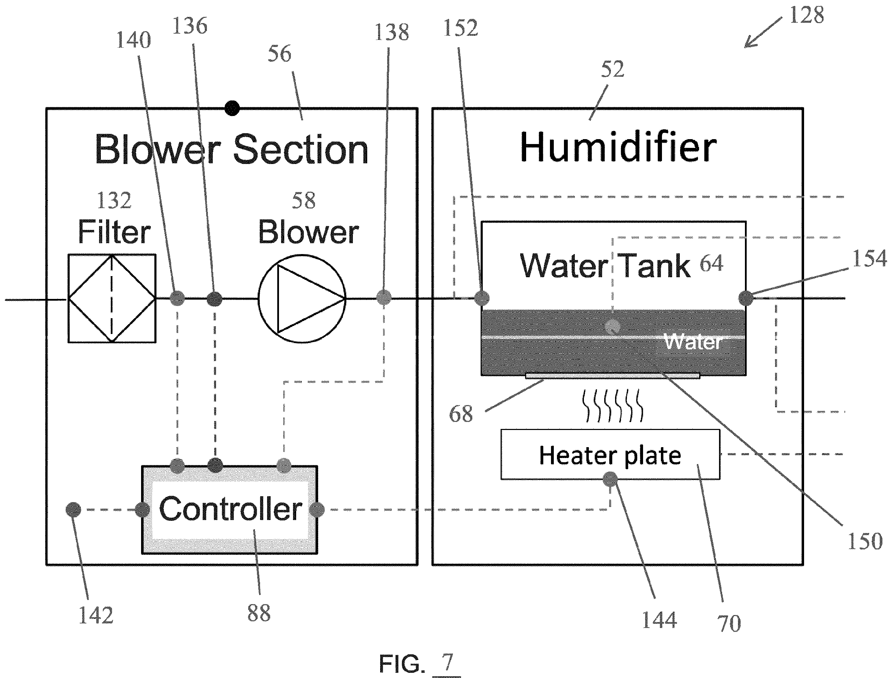

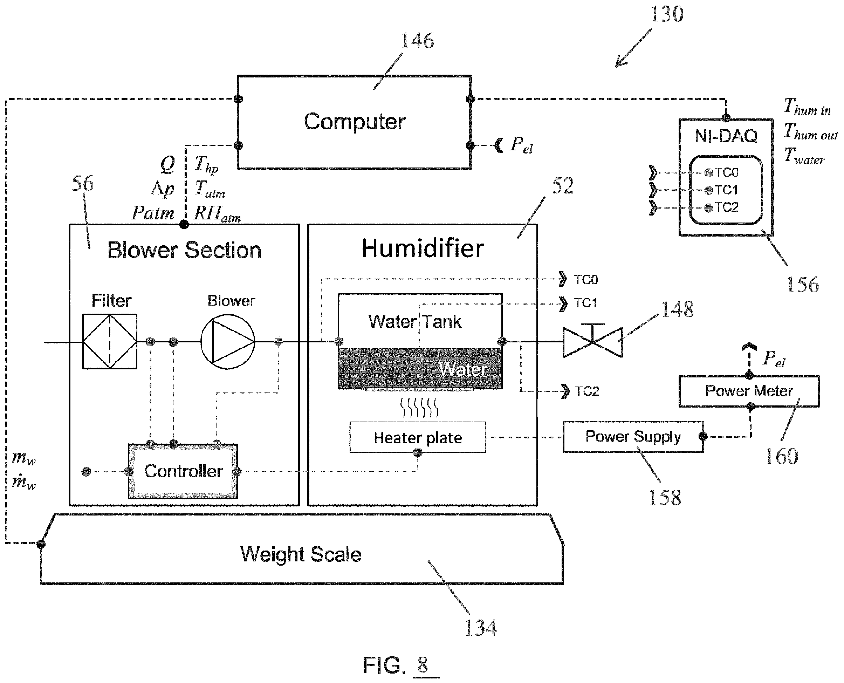

Referring now to Figures 7 and 8, block diagram schematic views 128 and 130, respectively, are shown of a humidifier set-up for use in conducting experiments as discussed herein according to embodiments of the present disclosure. The humidifier 52 of Figures 7 and 8 is similar to that of Figure 2, with the following additional components. An inlet filter 132 is used for filtering air received an air intake of the blower section, and filtered air is fed into the blower intake. In addition, a number of sensors are shown, wherein each sensor is configured to generate respective output signals conveying information that indicates one or more device parameters and other parameters. For example, an electronic weight scale 134 (e.g., Mettler Toledo MS6002SDR/01 NewClassic MF) is used for obtaining a measure of mass of the water $m_w$ (in units of mg), further used in determining an evaporation rate $\dot{m}_w$ (in units of mg/s). A flow sensor 136 is used to obtain a flow rate $Q$ (in units of L/min) at standard conditions (i.e., 1 bar absolute pressure and 20 °C temperature). A pressure sensor 138 is used to obtain a pressure measurement $\Delta p$ of the blower/fan (in units of $cmH_2O$). An ambient temperature and relative humidity sensor 140 is used for obtaining ambient temperature $T_{atm}$ (in units of °C) and relative humidity $RH_{atm}$ (in units of %). An atmospheric pressure sensor 142 is used to obtain ambient pressure $p_{atm}$ (in units of mbar). A heater plate temperature sensor 144 is used for obtaining a heater plate temperature $T_{hp}$ in units of °C

In the set-up shown in Figure 8, a computer 146 is used to receive each of the generated sensor output signals, via the controller 88, which include: (i) device parameters selected from the group consisting of: the heater plate temperature, the pressure of the breathable gas generated by the blower, and the flow rate of the breathable gas, and (ii) additional parameters of ambient temperature, ambient relative humidity, and ambient pressure.

Referring still to Figures 7 and 8, a pressurized flow of breathable gas input to the breathable gas inlet, humidified, and then output via the humidified breathable gas outlet. Coupled to the humidified breathable gas outlet is a flow regulator 148, for use in regulating a flow during experiments as discussed herein. The flow regulator 148 is used to control, at a specified pressure, the air flow rate through the humidifier 52. In addition, temperature sensors (i.e., thermocouples 150, 152, 154, respectively) are provided for measurement of water temperature ($T_w$) in units of [K], humidifier inlet temperature ($T_{hum\,in}$) in units of [K], and humidifier outlet temperature ($T_{hum\,out}$) in units of [K]. Each of these three temperature sensors are coupled to respective inputs of a thermocouple input module 156 (e.g., a National Instruments™ Model NI 9211) which includes delta-sigma analog-to-digital converters for converting the thermocouple signals into temperature signal measurements. Figure 8 further illustrates a power supply 158 and a power meter 160 for measuring an output power ($P_{el}$) in units of Watts provided to the heater plate by the power supply.

As discussed herein, controller 88 is configured to control an evaporation rate of the volume of water 66 housed in the water reservoir 62 with an adaptive electrical power input control according to a power control algorithm for humidifying the flow of breathable gas 60 received at the breathable gas inlet 72 of the water reservoir 62 into a flow of humidified breathable gas 78 at the humidified breathable gas outlet 74 of the water reservoir 62.

To demonstrate the principle of controlling an evaporation rate of the volume of water 66 housed in the water reservoir 62 with adaptive electrical power input control according to a power control algorithm of the present disclosure, we have executed eight (8) experiments. The experiments were conducted with the use of a respiratory therapy device (i.e., a DS1 DreamStation™ (DS1) system). The evaporation rate was measured with an electronic mass balance (i.e., weight scale). The ambient temperature, ambient pressure and the relative humidity were measured but not controlled.

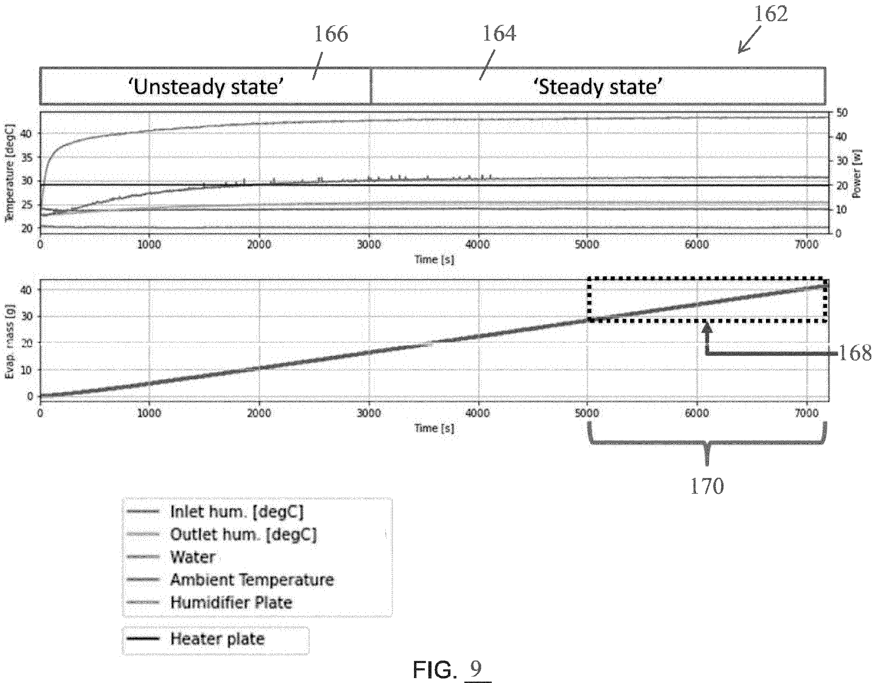

**Figure 9** is plot 162 of recorded parameters in a detailed example of a measurement run at one setting of a DOE. All relevant parameters were recorded just after steady state 164 is reached, which takes some time, as shown in Figure 9. Note that we see an initially rapid increase of the heater plate temperature (e.g., within the first 1000 seconds) in the 'unsteady state' 166, while the water temperature slowly increases (e.g., within the first 3000 seconds) to the steady state values. The evaporation rate is calculated by performing linear regression on the evaporated mass, during steady state 168. In one embodiment, steady state results are determined by averaging over the last 2000 seconds, as indicated by reference numeral 170.

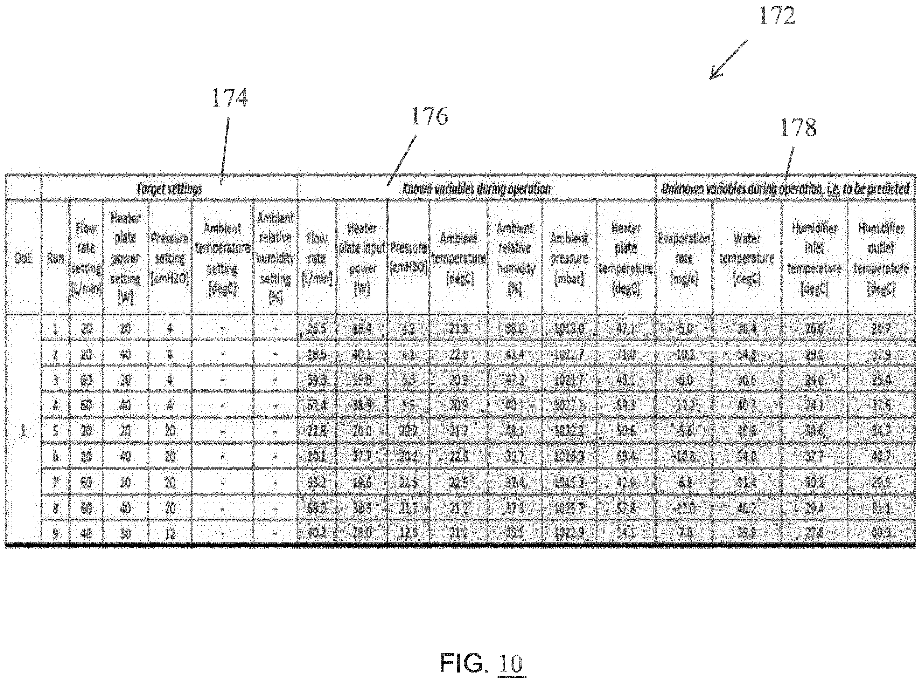

Referring now to **Figure 10,** a table 172 is shown of results of a first DOE ($DOE_1$) with variation of flow rate setting *(Q),* pressure setting ($\Delta p$)*,* and heater plate power setting ($P_{el}$). Table 172 lists target settings 174, known variables during operation 176, and unknown variables during operation (i.e., to be predicted) 178. In particular, the experimental results include eight measurements at extreme settings of $\Delta p$, $Q$ and $P_{el}$ and an extra measured centre point at a central location in the parameter space. It is noted that the Gage Repeatability and Reproducibility (gage R&R) of the measurement system has been verified and is good. Gage R&R is a methodology used to define the amount of variation in the measurement data due to the measurement system. It then compares measurement variation to the total variability observed, consequently defining the capability of the measurement system.

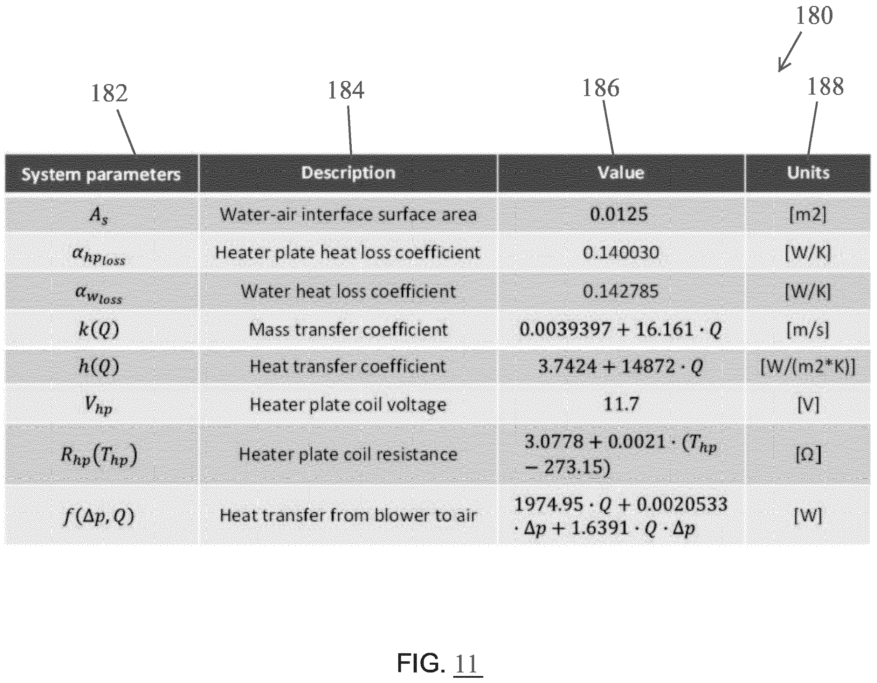

**Figure 11** is a table 180 of system parameters determined from the first DOE ($DOE_1$) and includes system parameters 182, descriptions 184, values 186, and units 188. From these nine (9) measurements (i.e., runs of the experimental results) we can determine from the four (4) equations (Eq. 7-10), the six (6) unknown system variables where the total error has been minimized. Figure 11 shows system parameters, description, value and units. All units are metric, e.g., Pa, $m^3/s$ and K. Note that it is also possible to determine the transfer function from somewhat less measurements, but preferably we select a minimum of parameters over the full parameter space.

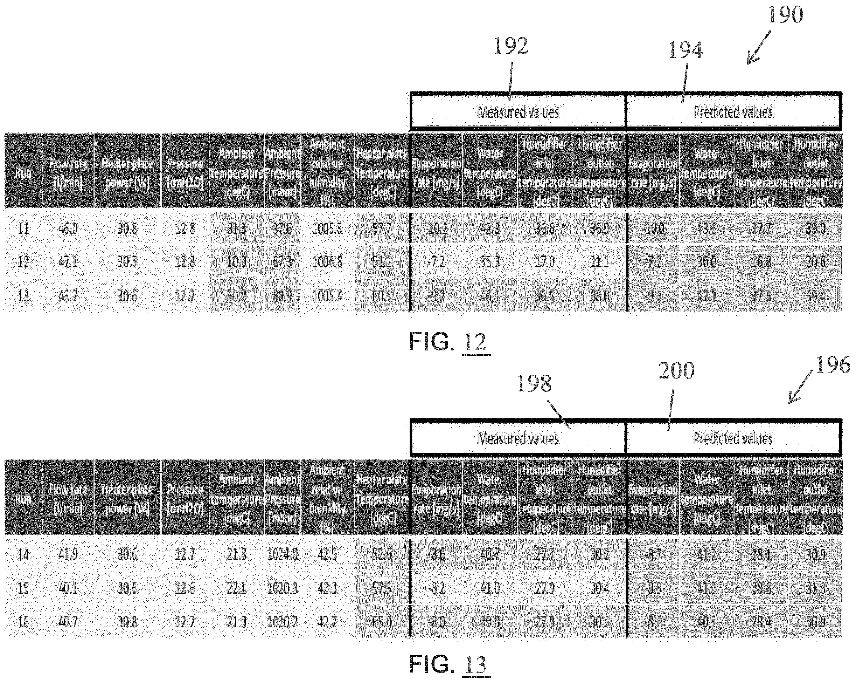

As a further verification, we have verified the influence of different environmental conditions, by varying the air temperature as well as the relative humidity. Therefore, we have executed similar experiments as for DOE 1 but now in a climate chamber, where temperature and relative humidity can be varied, for a second DOE (DOE 2). We have also verified that the results in the lab and in the climate chamber are identical at identical temperature and relative humidity settings. In principle, there is no need to execute this as its behavior is known from (Eq. 7-10), but it shows the strength of the heat and mass transfer model. Finally, we have used two deliberately different thermal contact[s] between the heater plate to verify that the heat and mass transfer model can still correctly predict the correct evaporation rate and humidifier outlet temperature. These results are executed in a third DOE (DOE 3). Figure 12 is a table 190 of measured and predicted values (192, 194, respectively) of experimental results of the second DOE (DOE 2) where ambient conditions have been varied. Figure 13 is a table 196 of measured and predicted values (198, 200, respectively) of experimental results of a third DOE (DOE 3) where ambient conditions and the thermal resistance of the heater plate have been varied.

The predicted values for DOE 2 and DOE 3 are very close to the measured values, especially for the evaporation rate, water temperature and humidifier inlet temperature, with an error of less than 4%, also the humidifier outlet temperature is very accurate with 5 of the 6 settings having an error less than 4% and error of 6%. The agreement between the prediction and experimental results for DOE 1 is also of similar accuracy, hence very good results.

Hence, with these DOE experiments, we show that we only need a small number of measurements and the heat and transfer model equation (Eq. 7-10) to accurately predict the four (4) output parameters (i.e., evaporation rate, water temperature, humidifier inlet temperature, and humidifier outlet temperature) over a large variation of the seven (7) input parameters (i.e., heater plate input power, heater plate electrical resistance, heater plate temperature, ambient pressure, ambient temperature, ambient relative humidity, and flow rate). Note that the function $f(\Delta p, Q)$ will depend on the fan or blower type that will be used. When multiple blower manufacturers will be used, the DOE may need to be carried out for each blower manufacturer.

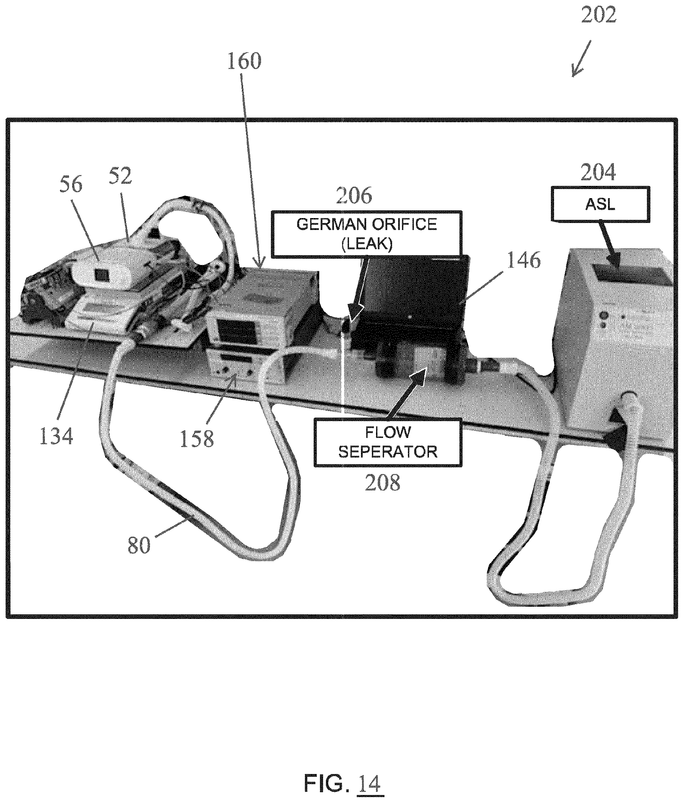

Turning now to **Figure 14,** a pictorial view is shown of an experimental set-up 202 with an automated servo lung (ASL) 204, which is an artificial lung, mimicking a patient for causing a varying flow rate through the humidifier 52 according to an embodiment of the present disclosure. In the set-up, a German orifice (leak) 206 and flow separator 208 were included in the patient interface tubing. The heater coil resistance was determined experimentally for the specific heater plate used in the experiment. We have also verified that it is reasonable to use an average flow rate. Therefore, we have measured the humidifier 52 with use of an ASL (i.e., automated servo lung breathing simulator), with a highly fluctuating air flow, representing the patient breathing and verified that all parameters measured with the dynamical system of Figure 14 are identical to the prediction based on the average flow rate through the ASL. All of the DOE, experimental measurements were done with a constant air flow rate (i.e., a constant air flow rate). In reality, a _varying_ air flow rate is delivered to the patient (i.e., during inhaling more air flow is delivered than during exhaling). The experiment with the artificial lung was done to prove that the evaporation rate _based on_ the average flow rate through the humidifier with such an artificial lung is _equal to_ our (i.e., predicted via the linear algorithm) steady state results operated at similar pressure and average flow rate.

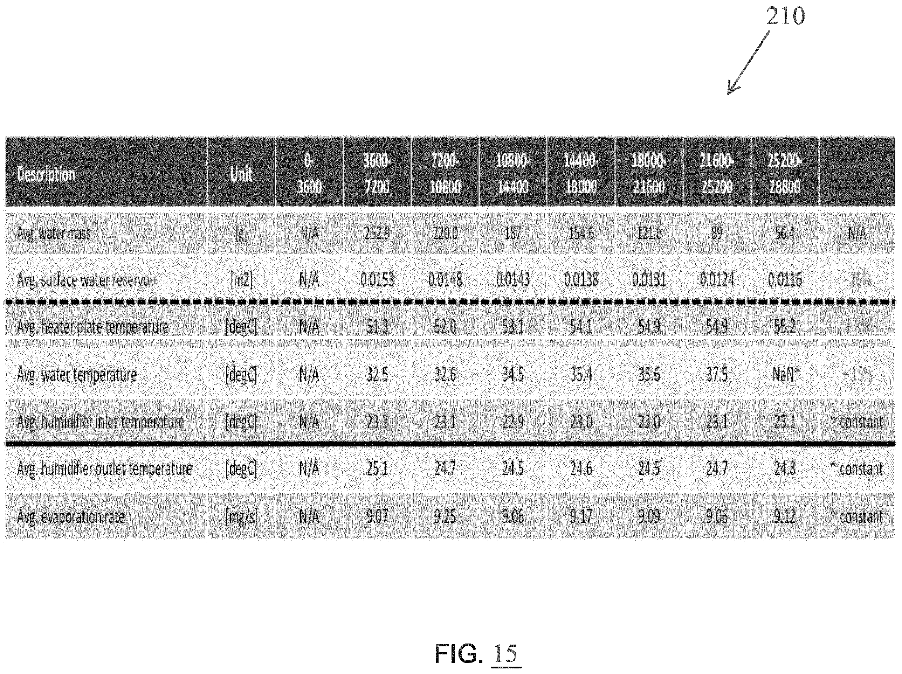

Further, we have also verified that the evaporation rate and humidifier inlet and outlet temperature remain constant when the water level is reduced. Figure 15 is a table 210 of experimental results showing that the evaporation rate and inlet and outlet humidifier temperatures remain constant with varying water level according to an embodiment of the present disclosure. Note that the water temperature is not constant due to the fact that the average contact area of the water with the air flow is reducing. In the heat and mass transfer model, the water temperature is determined when steady state is reached, i.e., in the second hour (see Figure 9).

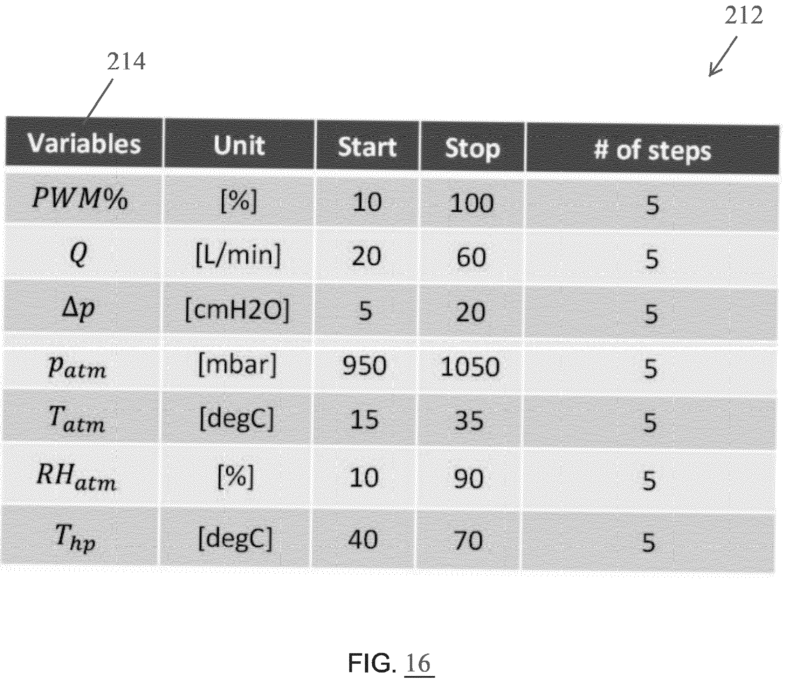

Now in principle, the steady state evaporation rate, humidifier inlet and outlet temperature and water temperature can be determined from solving the system of four (4) non-linear equations with four (4) unknowns (Eq. 7-10), now with the known system parameters. This is not very practical for a control algorithm; therefore, linear transfer functions were determined by solving a large number of variations of the seven (7) important input parameters (i.e., also referred to herein as input variables) for the heat and mass transfer model as shown in Figure 16. Figure 16 is a table 212 of variables 214 used in a variation scheme of seven input parameters to determine the linear transfer function according to an embodiment of the present disclosure. Note that all these input variables can be measured in the humidifier appliance. This would result in a total of 78125 simulations; however, these were limited to 21801 simulations to fulfil an effective thermal resistance $0.2 < R_{th} < 0.8$.

Each of the four (4) output parameters (evaporation rate, humidifier inlet and outlet temperature and water temperature) can be written as:

$$y_{pred} = c_0 + c_1 \cdot Q + c_2 \cdot \Delta p + c_3 \cdot PWM\% + c_4 \cdot T_{atm} + c_5 \cdot p_{atm} + c_6 \cdot RH_{atm} + c_7 \cdot T_{hp}$$

$$(\text{Eq. 11})$$

It appears that this simple linear regression function can very well describe the system of four (4) non-linear equations with four (4) unknowns (Eq. 7-10).

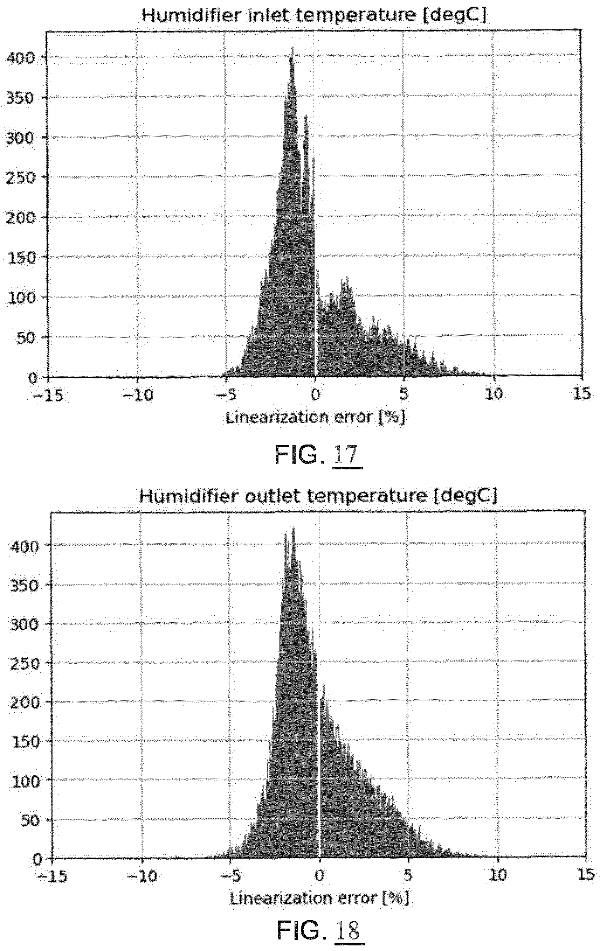

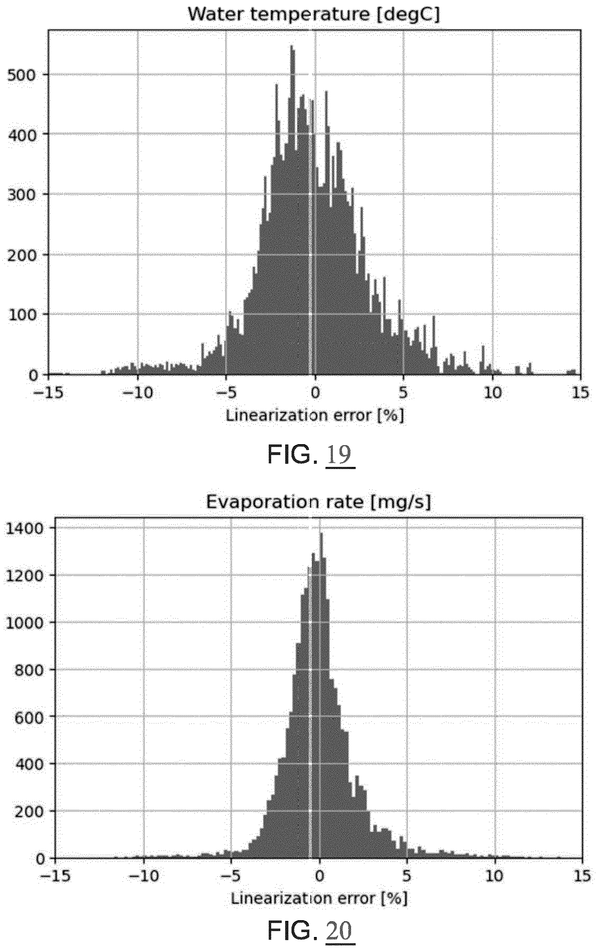

**Figures 17-20** are plots of linearization errors for humidifier inlet temperature (Figure 17), humidifier outlet temperature (Figure 18), water temperature (Figure 19), and evaporation rate (Figure 20), respectively, according to an embodiment of the present disclosure. The linearization errors are very small, especially for the most important parameter:

the evaporation rate (i.e., approximately $\pm$ 4%).

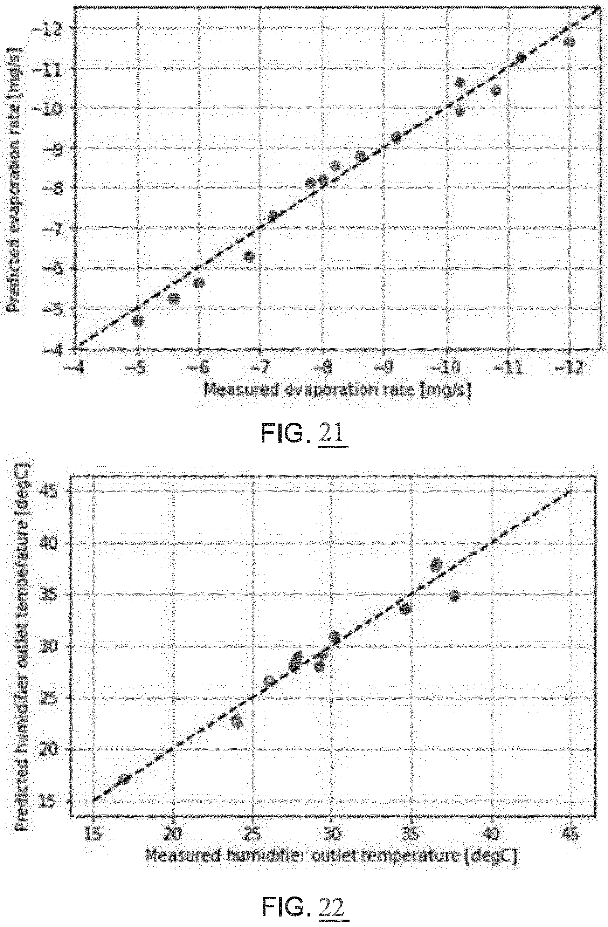

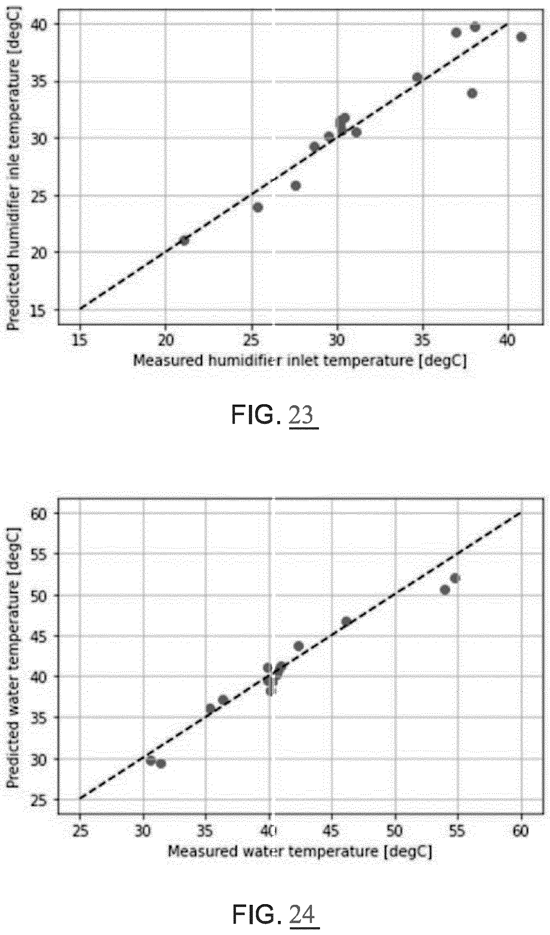

**Figures 21-24** are plots illustrating a comparison between all measurements and predictions of evaporation rate (Figure 21), humidifier outlet temperature (Figure 22), humidifier input temperature (Figure 23), and water temperature (Figure 24), respectively, based on the simplified linear transfer function according to an embodiment of the present disclosure. In particular, Figures 21-14 show the results of all 15 measurements and the linearized transfer function (Eq. 11) for each output parameter, showing a very small error for all measurements, including the ones with unknown thermal resistance.

Note that it is of course possible to extend the number of experiments to derive the system parameters and include measurements from DOE 2 and 3. This gives a slightly better prediction. In the final power control algorithm, the % PWM (as a representative for the electrical input power) should be determined as a function of the preferred evaporation rate. This follows immediately from (Eq. 11) and can be written (with the evaporation rate, $\dot{m}_w$) as:

$$PWM\% = 1/c_3 \cdot \dot{m}_w - c_0/c_3 - c_1/c_3 \cdot Q - c_2/c_3 \cdot \Delta p - c_4/c_3 \cdot T_{atm} - c_5/c_3 \cdot p_{atm} - c_6/c_3 \cdot RH_{atm} - c_7/c_3 \cdot T_{hp}$$

$$(\text{Eq. 12})$$

The values for the other three (3) parameters (i.e., water temperature, and humidifier inlet and outlet temperatures) can be determined from the other transfer functions (from Eq. 11) and the known PWM%.

It is important to realize that although the heater plate temperature $T_{hp}$ can be measured it is not a priori known what its steady state value will be, as this will depend on the unknown thermal heater plate resistance. Fortunately, the contribution of this term in (Eq. 12) appears to be very small, so we can start with an initial heater plate temperature $T_{hp,i}$, which is for instance the lowest steady state heater plate temperature, which follows from Figure 8 to be 43 °C. This will give us a starting PWM duty cycle of the heater plate coil, PWM%. As we measure the actual $T_{hp}$, we can correct the PWM% as soon as the actual $T_{hp}$ exceeds $T_{hp,i}$ and use the actual $T_{hp}$ in (Eq. 12). Then both $T_{hp}$ and PWM% will slowly increase until steady state has been reached, or reaches a predetermined percentage (e.g., 90%, 95%, or higher) of steady state. We will use a target setting for the PWM duty cycle of the heater plate coil (PWM%) increase over time to determine when steady state has been obtained. Preferably, the PWM% varies in a range of 0.01% - 5% per hour and more preferably in a range between 0.1-1% per hour. Once the steady state criterion has been reached, we will fix (i.e., set at a desired value) the PWM% signal.

Note that this procedure makes it possible to adapt the PWM% when different ambient conditions have been obtained or changes in the device condition occur. For instance, a change in mask leakage will require a change in flow rate and hence also a change in PWM%. The change in the PWM% is provided to maintain a fixed evaporation rate or relative humidity of the air entering a patient interface or mask. In addition, it is now possible to continuously set the required evaporation rate instead of the five (5) settings with respect to known humidifier devices (i.e., five (5) customer selectable settings for heater plate temperatures (from low to high heater plate temperature) as a function of mask pressure, average air flow rate through the fan, ambient temperature and relative humidity). Furthermore, it is also possible to adjust the evaporation rate to a certain relative humidity reaching the patient. Especially, for a heated tube this is possible as the temperature of the air reaching the patient interface or mask will be known. Then, it is possible to calculate from the ambient temperature and relative humidity the required evaporation rate.

Now, one of the important parameters will be the accuracy with which the power can be adjusted. In the experiments to derive the transfer functions (Eq. 11), we have controlled the electrical power to the heater plate very accurately, see Figure 8. One way to do this is to use rather tight specifications for the electrical resistance of the heater plate. However, this could be somewhat challenging for heater plate manufacturers, further, when heater plates from different manufacturers are used for a given humidifier implementation. There could be a variation in the mean electrical resistance of the heater plate(s). To address this concern with respect to variation in the mean electrical resistance of the heater plate(s), the specific mean electrical resistance (as a function of heater plate temperature) for respective heater plate(s) from each different heater plate manufacturer is included in the computer logic of the power control algorithm.

Another option to accurately know the electrical power delivered to the heater plate is by using a shunt resistance 92 in series with the heater plate, see Figure 2. When a known PWM% is used, the voltage drop over the shunt resistance 92 can be measured and, in this way, the electrical current to the heater plate 70 and with the known measured shunt resistor voltage and %PWM, the power (i.e., P=V·I) to the heater plate 70 can be determined accurately and independent of the exact heater plate electrical resistance.

Another method to measure the applied power to the heater plate is by using (Eq. 4) and the known thermal capacity of the heater plate, which is expected to show little variation from heater plate to heater plate. When we apply a little waiting time between the time the humidifier (and respiratory device) appliance is switched on and the time where power is applied to the heater plate, we know that the temperature of the heater plate is equal to the water temperature. Then it follows from (Eq. 4) that the electrical power can be obtained from the initial gradient in heater plate temperature.

From **Figure 9,** we learn that the water temperature takes some time (i.e., approximately 30 minutes or 1800 s) to reach steady state and that the initial evaporation rate, during the unsteady state, is lower than during the steady state. However, such a scenario may be uncomfortable for a patient. During this unsteady state, the applied power could be the power (or PWM%) that is predicted from the steady [i.e., steady state] linear transfer function (Eq. 12); however, it is also possible to increase the power input to the heater plate, such that the water heats up quicker.

The moment to adjust the heater plate power to the calculated steady state values can be determined by applying this larger power (i.e., initially during the unsteady state) for a fixed time of say 5 minutes (300 seconds), which is substantially lower than (e.g., on the order of between 15x to 25x lower than) the expected equilibrium time (e.g., 5000-7200 seconds or 83-120 minutes). Further this time setting can be improved by waiting a small time (e.g., 30-100 seconds) after the humidifier (and respiratory device) appliance is switched on as then the temperature of the heater plate will become equal to the initial water temperature. This heater plate temperature can be compared with the expected equilibrium temperature (i.e., at steady state) and the timing for the larger heating power can be estimated from this difference.

As a further improvement, the heater plate power can be switched off after a fixed heating time at larger power than the steady state power for some time. The temperature of the heater plate will drop to the water temperature after approximately 30-100 seconds, depending on the water level, and can be measured when the temperature of the heater plate has obtained steady state. As a still further improvement, it is possible to determine the cooling down curve of the heater plate and fit an exponential decay function through this curve. This makes it possible to estimate the water temperature even quicker.

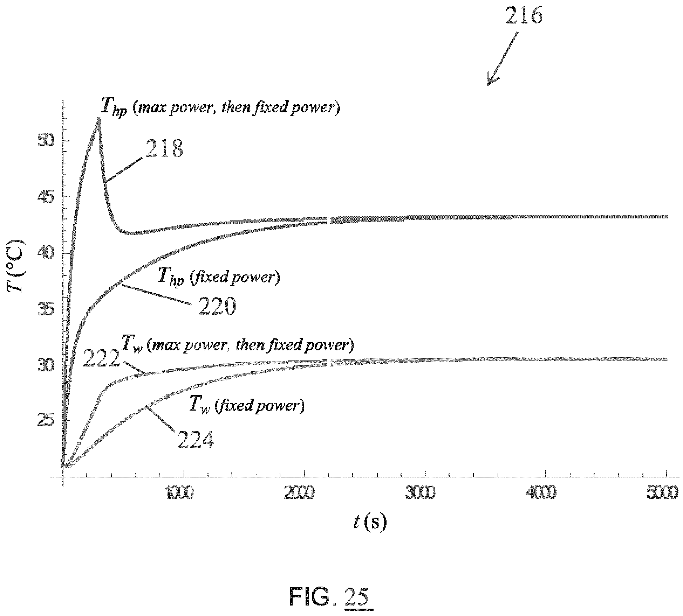

Referring now to **Figure 25,** a plot 216 is shown illustrating an example of power control with a maximum power according to an embodiment of the present disclosure. Curve 218 is representative of the temperature of the heater plate ($T_{hp}$) in response to the maximum power, then fixed power. Curve 220 is representative of the temperature of the heater plate ($T_{hp}$) in response to fixed power alone. Curve 222 is representative of the temperature of the volume of water ($T_w$) in response to the maximum power, then fixed power. Curve 224 is representative of the temperature of the volume of water ($T_w$) in response to fixed power alone.

In particular, the power control algorithm includes an accelerated steady state function for use in obtaining a steady state equilibrium faster than without the accelerated steady state function. The controller, via the power control algorithm, is configured to (i) initially set a power input to the heater plate to an increased power input compared to a quasi-steady state, and (ii) responsive to a detection of at least one of the generated sensor output signals attaining at least a predetermined percentage of its quasi-steady state value, switch to quasi-steady state power control algorithm power level corresponding to quasi-steady state settings.

With reference still to Figure 25, a substantially faster time to equilibrium is possible with this method according to an embodiment of the present disclosure. The example of power control with maximum power (as illustrated in Figure 25) is Run 3 from DOE 1, which has been calculated numerically. The first 300 seconds, we apply maximum power of 40 W and then switch back to 20 W. Indeed, it appears to be possible to substantially decrease the time to steady state (i.e., from 3000 seconds to 1000 seconds).

In another embodiment, the temperature of the heater plate is controlled via the power control algorithm such that it may never become larger than a given temperature to prevent heater plate temperatures rising above a given threshold maximum heater plate temperature. In particular, the heater plate temperature can be used to detect when the water volume in the reservoir or water tank has become reduced to very low levels (i.e., to detect a water volume below a given threshold minimum allowable or safe volume or level). The heater plate temperature will then increase (i.e., in response to the water volume decreasing below the given threshold minimum volume or level). It is possible to give a consumer a warning, or to (gradually) reduce the power to the heater plate until the power transfer has fully stopped in order to prevent an undesirable situation in that the heater plate temperature will increase to too high values of temperature. Preferably this is done (i.e., power to the heater plate is reduced) when the heater plate temperature is more than 5-50 °C above its first steady state value, and more particularly, when the heater plate temperature is more than 10-25 °C above its first steady state value.

As discussed herein, a power control algorithm is adapted to control the evaporation rate of a humidifier in a sleep or respiratory device. An electric power input is used as a control parameter to regulate the evaporation rate. A linear transfer function has been obtained where the required power input can be described as a function of the desired evaporation rate, relevant humidifier and respiratory device parameters: the heater plate temperature, the average pressure generated by the blower, the average flow rate and the ambient temperature, relative humidity, and ambient pressure. The power control algorithm has several advantages over the current method with respect to known humidifier devices. The power control algorithm has been determined to be very accurate. The evaporation rate can be continuously adapted, including an adaptation to different environmental conditions. In addition, the evaporation rate is not sensitive to the contact between the heater plate and water tank, including scale accumulation on the metal plate of the water tank. Furthermore, the evaporation rate is independent of the water volume in the water tank. The power control algorithm is easily implemented in control logic and for each new humidifier design, the linear transfer function requires only a few experiments to adapt the linear transfer function to each respective new humidifier design.

Although only a few exemplary embodiments have been described in detail above, those skilled in the art will readily appreciate that many modifications are possible in the exemplary embodiments without materially departing from the novel teachings and advantages of the embodiments of the present disclosure. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

**Claims**

1. A humidifier (12) of a heated pass-over type for use in a sleep or respiratory therapy device (54) that includes a blower section (26) having a blower for supplying a pressurized flow of breathable gas, comprising:

   a heater plate (70);
   a water reservoir (62) structured to house a volume of water (66), the water reservoir (62) having a breathable gas inlet (72) and a humidified breathable gas outlet (74), wherein the water reservoir (62) includes at least one surface for contacting the heater plate (70); and
   one or more sensors (86) configured to generate output signals conveying information about an operating status of the humidifier (12) and ambient conditions;
   a controller (88) configured to control an evaporation rate of the volume of water housed in the water reservoir (62) with a power control according to a power control algorithm for humidifying the flow of breathable gas received at the breathable gas inlet of the water reservoir (62) into a flow of humidified breathable gas at the humidified breathable gas outlet of the water reservoir (62), wherein the power control algorithm includes a transfer function in which a required power input to the heater plate (70) is a function of a desired evaporation rate based upon generated sensor output signals,
   **characterized in that**
   the power control algorithm includes an accelerated steady state function for use in obtaining a steady state equilibrium faster than without the accelerated steady state function,
   wherein the controller (88), via the power control algorithm, is configured to (i) initially set a power input to the heater plate (70) to an increased power input compared to a quasi-steady state, and (ii) responsive to a detection of at least one of the generated sensor output signals attaining at least a predetermined percentage of its quasi-steady state value, switch to quasi-steady state power control algorithm power level corresponding to quasi-steady state settings,
   wherein the controller (88) is configured to:

   apply the increased power input for a fixed time,
   wait an amount of time as a temperature of the heater plate (70) becomes equal to a temperature of the water, and
   estimate a timing for the increased power input from a difference between the temperature of the heater plate (70) and an expected temperature of the steady state equilibrium.

2. The humidifier (12) according to claim 1, wherein the generated sensor output signals are selected from a device parameter group consisting of heater plate (70) temperature, a pressure generated by the blower, and a flow rate generated by the blower, and an ambient parameters group consisting of ambient temperature, ambient relative humidity, and ambient pressure.

3. The humidifier (12) according to claim 1, wherein the transfer function is specific to a given humidifier design and where the required power input to the heater plate (70) during a quasi-steady state can be described as a function of (i) a desired quasi-steady state evaporation rate and (ii) generated sensor output signals.

4. The humidifier (12) according to claim 3, wherein the generated sensor output signals include:

   (i) device parameters selected from the group consisting of: a heater plate temperature, a pressure of the breathable gas generated by the blower, and a flow rate of the breathable gas, and
   (ii) additional parameters of ambient temperature, ambient relative humidity, and ambient pressure.

5. The humidifier (12) according to claim 1, wherein the electrical input power, represented as a variable Pow, is determined, via the power control algorithm, as a function of the desired evaporation rate given by a linearized transfer function:

$$Pow = c_0 + c_1 \cdot \dot{m}_w + c_2 \cdot Q + c_3 \cdot \Delta p + c_4 \cdot T_{atm} + c_5 \cdot p_{atm} + c_6 \cdot RH_{atm} + c_7 \cdot T_{hp},$$

   wherein

   $\dot{m}_w$ is evaporation rate in units of mg/s;
   $Q$ is flow rate in units of L/min at standard conditions;
   $\Delta p$ is pressure by the blower in units of $cmH_2O$;
   $T_{atm}$ is ambient temperature in units of °C;
   $p_{atm}$ is ambient pressure in units of mbar;
   $RH_{atm}$ is relative humidity in units of %;
   $T_{hp}$ is heater plate temperature in units of °C; and
   $c_0$, $c_1$, $c_2$, $c_3$, $c_4$, $c_5$, $c_6$, and $c_7$ are predetermined coefficients.

6. The humidifier (12) according to claim 5, wherein values of water temperature of the volume of water housed in the water reservoir (62), inlet humidifier temperature of the flow of breathable gas at the breathable gas inlet (72), and outlet humidifier temperature at the humidified breathable gas outlet (74) are each determined using a respective linearized transfer function:

$$y_{pred} = c_0 + c_1 \cdot Q + c_2 \cdot \Delta p + c_3 \cdot Pow + c_4 \cdot T_{atm} + c_5 \cdot p_{atm} + c_6 \cdot RH_{atm} + c_7 \cdot T_{hp}.$$

7. The humidifier (12) according to claim 1, wherein the water reservoir (62) includes a plate disposed on or integral with the at least one surface of the water reservoir (62) and configured for being placed in contact with the heater plate (70).

8. The humidifier (12) according to claim 1, wherein the required power input comprises an electric power input to the heater plate (70) that is operable as a control parameter to regulate the evaporation rate.

9. The humidifier (12) according to claim 1, wherein the required electrical power input to the heater plate (70) comprises an electrical power controlled by pulse width modulation.

10. The humidifier (12) according to claim 1, wherein the evaporation rate is continuously adapted to changes in the operating status of the humidifier (12) and the ambient conditions.

11. The humidifier (12) according to claim 1, further comprising a shunt resistance (92) in series with the heater plate (70), wherein the controller (88) is further configured to obtain a measurement of electrical power delivered to the heater plate (70) via measuring a voltage drop across the shunt resistance (92), determining electrical current to the heater plate (70) using the measured voltage drop across the shunt resistance (92) and voltage, $V_{hp}$, across the heater plate (70).

12. The humidifier (12) according to claim 1, wherein the controller (88) is further configured, responsive to a detection of an increase in heater plate temperature above a threshold amount, to reduce the power input to the heater plate (70).

13. A gas delivery system for delivering a pressurized flow of humidified breathable gas to a patient via a patient circuit (32), comprising:

   a blower assembly having a blower adapted to generate the pressurized flow of breathable gas, and a gas flow path including an inlet and an outlet; and

a humidifier (12) according to claim 1, wherein there is a fluidic coupling between the blower, the humidifier (12), and the patient circuit.

14. A method of humidifying a flow of breathable gas in a sleep or respiratory therapy device (54) that includes a blower section (26) having a blower for supplying a pressurized flow of breathable gas using a humidifier (12) of a heated pass-over type, the method comprising:

providing a heater plate (70);

providing a water reservoir (62) structured to house a volume of water, the water reservoir (62) having a breathable gas inlet and a humidified breathable gas outlet, wherein the water reservoir (62) includes at least one surface configured for being in contact with the heater plate (70); and

providing one or more sensors configured to generate output signals conveying information that indicates one or more device parameters selected from the group consisting of heater plate temperature, pressure of the breathable gas, and flow rate of the breathable gas, and other parameters that comprise an ambient temperature, a relative humidity, and ambient pressure;

controlling, via a controller, an evaporation rate of the volume of water housed in the water reservoir (62) with a power control according to a power control algorithm for humidifying the flow of breathable gas received at the breathable gas inlet of the water reservoir (62) into a flow of humidified breathable gas at the humidified breathable gas outlet of the water reservoir (62), wherein the power control algorithm includes a transfer function in which a required power input to the heater plate (70) is a function of a desired evaporation rate based upon generated sensor output signals,

wherein the power control algorithm includes an accelerated steady state function for use in obtaining a steady state equilibrium faster than without the accelerated steady state function, the method further comprising:

initially setting, via the power control algorithm a power input to the heater plate (70) to an increased power input compared to a quasi-steady state, and

responsive to a detection of at least one of the generated sensor output signals attaining at least a predetermined percentage of its quasi-steady state value, switching to quasi-steady state power control algorithm power level corresponding to quasi-steady state settings,

applying the increased power input for a fixed time,

waiting an amount of time as a temperature of the heater plate (70) becomes equal to a temperature of the water,

estimating a timing for the increased power input from a difference between the temperature of the heater plate (70) and an expected temperature of the steady state equilibrium.

15. The method according to claim 14, wherein the generated sensor output signals are selected from a device parameter group consisting of heater plate temperature, a pressure generated by the blower, and a flow rate generated by the blower, and an ambient parameters group consisting of ambient temperature, ambient relative humidity, and ambient pressure.

**Patentansprüche**

1. Befeuchter (12) von einem beheizten Passover-Typ zur Verwendung in einer Schlaf- oder Atemtherapievorrichtung (54), die einen Gebläseabschnitt (26) beinhaltet, der ein Gebläse zum Zuführen eines druckbeaufschlagten Atemgasstroms aufweist, umfassend:

eine Heizplatte (70);

einen Wasserbehälter (62), der dazu strukturiert ist, ein Wasservolumen (66) aufzunehmen, wobei der Wasserbehälter (62) einen Atemgaseinlass (72) und einen Auslass (74) für befeuchtetes Atemgas aufweist, wobei der Wasserbehälter (62) mindestens eine Oberfläche zum Kontaktieren der Heizplatte (70) beinhaltet; und

einen oder mehrere Sensoren (86), die dazu konfiguriert sind, Ausgangssignale zu erzeugen, die Informationen über einen Betriebszustand des Befeuchters (12) und Umgebungsbedingungen übermitteln;

eine Steuereinheit (88), die dazu konfiguriert ist, eine Verdunstungsrate des Wasservolumens, das im Wasserbehälter (62) aufgenommen ist, mit einer Leistungssteuerung gemäß einem Leistungssteuerungsalgorithmus zu steuern, um den Atemgasstrom, der am Atemgaseinlass des Wasserbehälters (62) empfangen wird, in einen Strom befeuchteten Atemgases am Auslass für befeuchtetes Atemgas des Wasserbehälters (62) zu befeuchten, wobei der Leistungssteuerungsalgorithmus eine Übertragungsfunktion beinhaltet, bei der eine erforderliche Leistungsaufnahme an der Heizplatte (70) eine Funktion einer gewünschten Verdunstungsrate basierend auf erzeugten Sensorausgangssignalen ist,

**dadurch gekennzeichnet, dass**

der Leistungssteuerungsalgorithmus eine beschleunigte Steady-State-Funktion beinhaltet, die dazu verwendet wird, ein stationäres Gleichgewicht schneller zu erreichen als ohne die beschleunigte Steady-State-Funktion, wobei die Steuereinheit (88) über den Leistungssteuerungsalgorithmus dazu konfiguriert ist, (i) zunächst eine Leistungsaufnahme an der Heizplatte (70) auf eine im Vergleich zu einem quasistationären Zustand erhöhte Leistungsaufnahme einzustellen und (ii) als Reaktion auf eine Erkennung, dass mindestens eines der erzeugten Sensorausgangssignale mindestens einen vorbestimmten Prozentsatz seines quasistationären Werts erreicht, auf einen Leistungspegel des quasistationären Leistungssteuerungsalgorithmus umzuschalten, der den quasistationären Einstellungen entspricht,

wobei die Steuereinheit (88) dazu konfiguriert ist:

die erhöhte Leistungsaufnahme für eine festgelegte Zeit anwenden,
eine Zeitspanne zu warten, bis eine Temperatur der Heizplatte (70) der Wassertemperatur entspricht, und
einen Zeitpunkt für die erhöhte Leistungsaufnahme aus einer Differenz zwischen der Temperatur der Heizplatte (70) und einer erwarteten Temperatur des stationären Gleichgewichts zu schätzen.

2. Befeuchter (12) nach Anspruch 1, wobei die erzeugten Sensorausgangssignale aus einer Vorrichtungsparametergruppe ausgewählt werden, die aus der Temperatur der Heizplatte (70), einem vom Gebläse erzeugten Druck und einer vom Gebläse erzeugten Durchflussrate besteht, sowie aus einer Umgebungsparametergruppe, die aus der Umgebungstemperatur, der relativen Umgebungsfeuchtigkeit und dem Umgebungsdruck besteht.

3. Befeuchter (12) nach Anspruch 1, wobei die Übertragungsfunktion spezifisch für eine bestimmte Befeuchterkonstruktion ist und wobei die erforderliche Leistungsaufnahme an der Heizplatte (70) während eines quasistationären Zustands als Funktion (i) einer gewünschten Verdunstungsrate im quasistationären Zustand und (ii) der erzeugten Sensorausgangssignale beschrieben werden kann.

4. Befeuchter (12) nach Anspruch 3, wobei die erzeugten Sensorausgangssignale Folgendes beinhalten:

(i) Vorrichtungsparameter, ausgewählt aus der Gruppe bestehend aus: einer Heizplattentemperatur, einem Druck des vom Gebläse erzeugten Atemgases und einer Durchflussrate des Atemgases, und
(ii) zusätzliche Parameter der Umgebungstemperatur, der relativen Umgebungsfeuchtigkeit und des Umgebungsdrucks.

5. Befeuchter (12) nach Anspruch 1, wobei die elektrische Eingangsleistung, dargestellt als Variable Pow, über den Leistungssteueralgorithmus als Funktion der gewünschten Verdunstungsrate bestimmt wird, die durch eine linearisierte Übertragungsfunktion gegeben ist:

$$Pow = c_0 + c_1 \cdot \dot{m}_w + c_2 \cdot Q + c_3 \cdot \Delta p + c_4 \cdot T_{atm} + c_5 \cdot p_{atm} + c_6 \cdot RH_{atm} + c_7 \cdot T_{hp},$$

wobei

$\dot{m}_w$ die Verdunstungsrate in Einheiten von mg/s ist;
$Q$ die Durchflussrate in Einheiten von L/min zu Standardbedingungen ist;
$\Delta p$ der Druck des Gebläses in Einheiten von cmH$_2$O ist;
$T_{atm}$ die Umgebungstemperatur in Einheiten von °C ist;
$p_{atm}$ der Umgebungsdruck in Einheiten von mbar ist;
$RH_{atm}$ die relative Luftfeuchtigkeit in Einheiten von % ist;
$T_{hp}$ die Heizplattentemperatur in Einheiten von °C ist; und
$c_0$, $c_1$, $c_2$, $c_3$, $c_4$, $c_5$, $c_6$ und $c_7$ vorgegebene Koeffizienten sind.

6. Befeuchter (12) nach Anspruch 5, wobei Werte der Wassertemperatur des im Wasserbehälter (62) aufgenommenen Wasservolumens, der Einlassbefeuchtertemperatur des Atemgasstroms am Atemgaseinlass (72) und der Auslassbefeuchtertemperatur am Auslass für befeuchtetes Atemgas (74) jeweils unter Verwendung einer entsprechenden linearisierten Übertragungsfunktion bestimmt werden:

$$y_{pred} = c_0 + c_1 \cdot Q + c_2 \cdot \Delta\mathrm{p} + c_3 \cdot Pow + c_4 \cdot T_{atm} + c_5 \cdot p_{atm} + c_6 \cdot RH_{atm} + c_7 \cdot T_{hp}.$$

7. Befeuchter (12) nach Anspruch 1, wobei der Wasserbehälter (62) eine Platte beinhaltet, die auf der mindestens einen Oberfläche des Wasserbehälters (62) angeordnet ist oder mit dieser integriert ist und so konfiguriert ist, dass sie mit der Heizplatte (70) in Kontakt gebracht wird.

8. Befeuchter (12) nach Anspruch 1, wobei die erforderliche Leistungsaufnahme eine elektrische Leistungsaufnahme an der Heizplatte (70) umfasst, die als Steuerparameter zur Regulierung der Verdunstungsrate dient.

9. Befeuchter (12) nach Anspruch 1, wobei die erforderliche elektrische Leistungsaufnahme an der Heizplatte (70) eine durch Pulsweitenmodulation gesteuerte elektrische Leistung umfasst.

10. Befeuchter (12) nach Anspruch 1, wobei die Verdunstungsrate kontinuierlich an Änderungen des Betriebszustands des Befeuchters (12) und der Umgebungsbedingungen angepasst wird.

11. Befeuchter (12) nach Anspruch 1, der weiter einen Shunt-Widerstand (92) in Reihe mit der Heizplatte (70) umfasst, wobei die Steuereinheit (88) weiter so konfiguriert ist, dass sie eine Messung der an die Heizplatte (70) gelieferten elektrischen Leistung durch Messen eines Spannungsabfalls über dem Shunt-Widerstand (92) erhält und den elektrischen Strom zur Heizplatte (70) unter Verwendung des gemessenen Spannungsabfalls über dem Shunt-Widerstand (92) und der Spannung $V_{hp}$ über der Heizplatte (70) bestimmt.

12. Befeuchter (12) nach Anspruch 1, wobei die Steuereinheit (88) weiter so konfiguriert ist, dass sie als Reaktion auf eine Erkennung eines Anstiegs der Heizplattentemperatur über einen Schwellenwert die Leistungsaufnahme an der Heizplatte (70) reduziert.

13. Gaszufuhrsystem zum Zuführen eines druckbeaufschlagten Stroms befeuchteten Atemgases zu einem Patienten über einen Patientenkreislauf (32), umfassend:

eine Gebläsebaugruppe, die ein Gebläse, das dazu geeignet ist, den druckbeaufschlagten Atemgasstrom zu erzeugen, und einen Gasströmungsweg, der einen Einlass und einen Auslass beinhaltet, aufweist; und einen Befeuchter (12) nach Anspruch 1, wobei zwischen dem Gebläse, dem Befeuchter (12) und dem Patientenkreislauf eine Fluidkopplung besteht.

14. Verfahren zum Befeuchten eines Atemgasstroms in einer Schlaf- oder Atemtherapievorrichtung (54), die einen Gebläseabschnitt (26) beinhaltet, der ein Gebläse zum Zuführen eines druckbeaufschlagten Atemgasstroms aufweist, unter Verwendung eines Befeuchters (12) von einem beheizten Passover-Typ, wobei das Verfahren umfasst:

Bereitstellen einer Heizplatte (70);
Bereitstellen eines Wasserbehälters (62), der dazu strukturiert ist, ein Wasservolumen aufzunehmen, wobei der Wasserbehälter (62) einen Atemgaseinlass und einen Auslass für befeuchtetes Atemgas aufweist, wobei der Wasserbehälter (62) mindestens eine Oberfläche beinhaltet, die dazu konfiguriert ist, mit der Heizplatte (70) in Kontakt gebracht zu werden; und
Bereitstellen eines oder mehrerer Sensoren, die so konfiguriert sind, dass sie Ausgangssignale erzeugen, die Informationen übermitteln, die einen oder mehrere Vorrichtungsparameter angeben, die aus der Gruppe ausgewählt sind, die aus der Temperatur der Heizplatte, dem Druck des Atemgases und der Durchflussrate des Atemgases sowie anderen Parametern besteht, die eine Umgebungstemperatur, eine relative Luftfeuchtigkeit und einen Umgebungsdruck umfassen;
Steuern, über eine Steuereinheit, einer Verdunstungsrate des im Wasserbehälter (62) aufgenommenen Wasservolumens mit einer Leistungssteuerung gemäß einem Leistungssteuerungsalgorithmus, um den Atemgasstrom, der am Atemgaseinlass des Wasserbehälters (62) empfangen wird, in einen Strom befeuchteten Atemgases am Auslass für befeuchtetes Atemgas des Wasserbehälters (62) zu befeuchten, wobei der Leistungssteuerungsalgorithmus eine Übertragungsfunktion beinhaltet, bei der eine erforderliche Leistungsaufnahme an der Heizplatte (70) eine Funktion einer gewünschten Verdunstungsrate basierend auf erzeugten Sensorausgangssignalen ist,
wobei der Leistungssteuerungsalgorithmus eine beschleunigte Steady-State-Funktion beinhaltet, die dazu verwendet wird, ein stationäres Gleichgewicht schneller zu erreichen als ohne die beschleunigte stationäre Funktion, wobei das Verfahren weiter umfasst:

anfängliches Einstellen einer Leistungsaufnahme an der Heizplatte (70) über den Leistungssteuerungsalgorithmus auf eine im Vergleich zu einem quasistationären Zustand erhöhte Leistungsaufnahme, und als Reaktion auf eine Erkennung mindestens eines der erzeugten Sensorausgangssignale, die mindestens einen vorbestimmten Prozentsatz ihres quasistationären Werts erreichen, Umschalten auf einen Leistungspegel des quasistationären Leistungssteuerungsalgorithmus, der den quasistationären Einstellungen entspricht,

Anlegen der erhöhten Leistungsaufnahme für eine festgelegte Zeit,

Warten einer Zeitspanne, bis eine Temperatur der Heizplatte (70) der Wassertemperatur entspricht,

Schätzen eines Zeitpunkts für die erhöhte Leistungsaufnahme aus einer Differenz zwischen der Temperatur der Heizplatte (70) und einer erwarteten Temperatur des stationären Gleichgewichts.

**15.** Verfahren nach Anspruch 14, wobei die erzeugten Sensorausgangssignale aus einer Vorrichtungsparametergruppe ausgewählt werden, die aus der Heizplattentemperatur, einem vom Gebläse erzeugten Druck und einer vom Gebläse erzeugten Durchflussrate besteht, sowie aus einer Umgebungsparametergruppe, die aus der Umgebungstemperatur, der relativen Umgebungsfeuchtigkeit und dem Umgebungsdruck besteht.

## Revendications

**1.** Humidificateur (12) de type pass-over chauffé à utiliser dans un dispositif de thérapie du sommeil ou respiratoire (54) qui inclut une section de soufflante (26) présentant une soufflante pour fournir un flux pressurisé de gaz respirable, comprenant :

une plaque chauffante (70) ;

un réservoir d'eau (62) structuré pour contenir un volume d'eau (66), le réservoir d'eau (62) présentant une admission de gaz respirable (72) et une évacuation de gaz respirable humidifié (74), dans lequel le réservoir d'eau (62) inclut au moins une surface destinée à entrer en contact avec la plaque chauffante (70) ;

un ou plusieurs capteurs (86) configurés pour générer des signaux de sortie transportant des informations sur un état de fonctionnement de l'humidificateur (12) et des conditions ambiantes ;

un dispositif de commande (88) configuré pour commander un taux d'évaporation du volume d'eau contenu dans le réservoir d'eau (62) avec une commande de puissance selon un algorithme de commande de puissance pour humidifier le flux de gaz respirable reçu au niveau de l'admission de gaz respirable du réservoir d'eau (62) et en faire un flux de gaz respirable humidifié au niveau de l'évacuation de gaz respirable humidifié du réservoir d'eau (62), dans lequel l'algorithme de commande de puissance inclut une fonction de transfert dans laquelle une entrée de puissance requise vers la plaque chauffante (70) dépend d'un taux d'évaporation souhaité basé sur des signaux de sortie de capteurs générés,

**caractérisé en ce que**

l'algorithme de commande de puissance inclut une fonction d'état stable accélérée à utiliser pour obtenir un équilibre à l'état stable plus rapidement que sans la fonction d'état stable accélérée,

dans lequel le dispositif de commande (88), via l'algorithme de commande de puissance, est configuré pour (i) régler initialement une entrée de puissance vers la plaque chauffante (70) sur une entrée de puissance accrue par rapport à un état quasi-stable, et (ii) en réponse à une détection d'au moins un des signaux de sortie de capteurs générés atteignant au moins un pourcentage prédéterminé de sa valeur d'état quasi-stable, passer à un niveau de puissance d'algorithme de commande de puissance d'état quasi-stable correspondant à des réglages d'état quasi-stable,

dans lequel le dispositif de commande (88) est configuré pour :

appliquer l'entrée de puissance accrue pendant une durée fixe,

attendre pendant un certain temps qu'une température de la plaque chauffante (70) devienne égale à une température de l'eau, et

estimer une temporisation pour l'entrée de puissance accrue à partir d'une différence entre la température de la plaque chauffante (70) et une température attendue de l'équilibre à l'état stable.

**2.** Humidificateur (12) selon la revendication 1, dans lequel les signaux de sortie de capteurs générés sont sélectionnés parmi un groupe de paramètres de dispositif constitué d'une température de plaque chauffante (70), une pression générée par la soufflante et un débit généré par la soufflante, et un groupe de paramètres ambiants constitué d'une température ambiante, une humidité relative ambiante et une pression ambiante.

**3.** Humidificateur (12) selon la revendication 1, dans lequel la fonction de transfert est spécifique à une conception d'humidificateur donnée et dans lequel l'entrée de puissance requise vers la plaque chauffante (70) pendant un état quasi-stable peut être décrite comme dépendant (i) d'un taux d'évaporation à l'état quasi-stable souhaité et (ii) de signaux de sortie de capteurs générés.

**4.** Humidificateur (12) selon la revendication 3, dans lequel les signaux de sortie de capteurs générés incluent :

(i) des paramètres de dispositif sélectionnés dans le groupe consistant en : une température de plaque chauffante, une pression du gaz respirable générée par la soufflante et un débit du gaz respirable, et
(ii) des paramètres supplémentaires de température ambiante, d'humidité relative ambiante et de pression ambiante.

**5.** Humidificateur (12) selon la revendication 1, dans lequel la puissance électrique d'entrée, représentée comme une variable Pow, est déterminée, via l'algorithme de commande de puissance, en fonction du taux d'évaporation souhaité donné par une fonction de transfert linéarisée :

$$Pow = c_0 + c_1 \cdot \dot{m}_w + c_2 \cdot Q + c_3 \cdot \Delta p + c_4 \cdot T_{atm} + c_5 \cdot p_{atm} + c_6 \cdot RH_{atm} + c_7 \cdot T_{hp},$$

dans laquelle

$\dot{m}_w$ est le taux d'évaporation en unités de mg/s ;
Q est le débit en unités de l/min dans des conditions standard ;
$\Delta p$ est la pression par la soufflante en unités de $cmH_2O$ ;
$T_{\alpha tm}$ est la température ambiante en unités de °C ;
$p_{atm}$ est la pression ambiante en unités de mbar ;
$RH_{atm}$ est l'humidité relative en unités de % ;
$T_{hp}$ est la température de plaque chauffante en unités de °C ; et
$c_0$, $c_1$, $c_2$, $c_3$, $c_4$, $c_5$, $c_6$ et $c_7$ sont des coefficients prédéterminés.

**6.** Humidificateur (12) selon la revendication 5, dans lequel les valeurs de température d'eau du volume d'eau contenu dans le réservoir d'eau (62), de température d'humidificateur d'admission du flux de gaz respirable au niveau de l'admission de gaz respirable (72) et de température d'humidificateur d'évacuation au niveau de l'évacuation de gaz respirable humidifié (74) sont déterminées chacune à l'aide d'une fonction de transfert linéarisée respective :

$$y_{pred} = c_0 + c_1 \cdot Q + c_2 \cdot \Delta p + c_3 \cdot Pow + c_4 \cdot T_{atm} + c_5 \cdot p_{atm} + c_6 \cdot RH_{atm} + c_7 \cdot T_{hp}.$$

**7.** Humidificateur (12) selon la revendication 1, dans lequel le réservoir d'eau (62) inclut une plaque disposée sur ou solidaire de la au moins une surface du réservoir d'eau (62) et configurée pour être placée en contact avec la plaque chauffante (70).

**8.** Humidificateur (12) selon la revendication 1, dans lequel l'entrée de puissance requise comprend une entrée de puissance électrique vers la plaque chauffante (70) qui peut être utilisée comme paramètre de commande pour réguler le taux d'évaporation.

**9.** Humidificateur (12) selon la revendication 1, dans lequel l'entrée de puissance électrique requise vers la plaque chauffante (70) comprend une puissance électrique commandée par une modulation d'impulsions en durée.

**10.** Humidificateur (12) selon la revendication 1, dans lequel le taux d'évaporation est adapté en continu aux changements de l'état de fonctionnement de l'humidificateur (12) et des conditions ambiantes.

**11.** Humidificateur (12) selon la revendication 1, comprenant en outre une résistance shunt (92) en série avec la plaque chauffante (70), dans lequel le dispositif de commande (88) est en outre configuré pour obtenir une mesure de puissance électrique délivrée à la plaque chauffante (70) via la mesure d'une chute de tension aux bornes de la résistance shunt (92), déterminant un courant électrique vers la plaque chauffante (70) à l'aide de la chute de tension mesurée aux bornes de la résistance shunt (92) et d'une tension, $V_{hp}$, aux bornes de la plaque chauffante (70).

**12.** Humidificateur (12) selon la revendication 1, dans lequel le dispositif de commande (88) est en outre configuré, en réponse à une détection d'une augmentation de la température de plaque chauffante au-dessus d'une quantité seuil, pour réduire l'entrée de puissance vers la plaque chauffante (70).

**13.** Système d'administration de gaz permettant d'administrer un flux pressurisé de gaz respirable humidifié à un patient via un circuit patient (32), comprenant :

un ensemble de soufflante présentant une soufflante adaptée pour générer le flux pressurisé de gaz respirable et un trajet de flux de gaz incluant une admission et une évacuation ; et
un humidificateur (12) selon la revendication 1, dans lequel est situé un couplage fluidique entre la soufflante, l'humidificateur (12) et le circuit patient.

**14.** Procédé d'humidification d'un flux de gaz respirable dans un dispositif de thérapie du sommeil ou respiratoire (54) qui inclut une section de soufflante (26) présentant une soufflante pour fournir un flux pressurisé de gaz respirable à l'aide d'un humidificateur (12) de type pass-over chauffé, le procédé comprenant :

la fourniture d'une plaque chauffante (70) ;
la fourniture d'un réservoir d'eau (62) structuré pour contenir un volume d'eau, le réservoir d'eau (62) présentant une admission de gaz respirable et une évacuation de gaz respirable humidifié, dans lequel le réservoir d'eau (62) inclut au moins une surface configurée pour être en contact avec la plaque chauffante (70) ; et
la fourniture d'un ou plusieurs capteurs configurés pour générer des signaux de sortie transportant des informations qui indiquent un ou plusieurs paramètres de dispositif sélectionnés dans le groupe constitué d'une température de plaque chauffante, une pression du gaz respirable et un débit du gaz respirable, et d'autres paramètres qui comprennent une température ambiante, une humidité relative et une pression ambiante;
la commande, via un dispositif de commande, d'un taux d'évaporation du volume d'eau contenu dans le réservoir d'eau (62) avec une commande de puissance selon un algorithme de commande de puissance pour humidifier le flux de gaz respirable reçu au niveau de l'admission de gaz respirable du réservoir d'eau (62) et en faire un flux de gaz respirable humidifié au niveau de l'évacuation de gaz respirable humidifié du réservoir d'eau (62), dans lequel l'algorithme de commande de puissance inclut une fonction de transfert dans laquelle une entrée de puissance requise vers la plaque chauffante (70) dépend d'un taux d'évaporation souhaité basé sur des signaux de sortie de capteurs générés,
dans lequel l'algorithme de commande de puissance inclut une fonction d'état stable accélérée à utiliser pour obtenir un équilibre à l'état stable plus rapidement que sans la fonction d'état stable accélérée, le procédé comprenant en outre :

le réglage initial, via l'algorithme de commande de puissance, d'une entrée de puissance vers la plaque chauffante (70) sur une entrée de puissance accrue par rapport à un état quasi-stable, et
en réponse à une détection d'au moins un des signaux de sortie de capteurs générés atteignant au moins un pourcentage prédéterminé de sa valeur d'état quasi-stable, le passage à un niveau de puissance d'algorithme de commande de puissance d'état quasi-stable correspondant à des réglages d'état quasi-stable, l'application de l'entrée de puissance accrue pendant une durée fixe,
l'attente pendant un certain temps qu'une température de la plaque chauffante (70) devienne égale à une température de l'eau,
l'estimation d'une temporisation pour l'entrée de puissance accrue à partir d'une différence entre la température de la plaque chauffante (70) et une température attendue de l'équilibre à l'état stable.

**15.** Procédé selon la revendication 14, dans lequel les signaux de sortie de capteurs générés sont sélectionnés parmi un groupe de paramètres de dispositif constitué d'une température de plaque chauffante, une pression générée par la soufflante et un débit généré par la soufflante, et un groupe de paramètres ambiants constitué d'une température ambiante, une humidité relative ambiante et une pression ambiante.

FIG. 1

FIG. 2

52
HUMIDIFIER

Water Reservoir 62

$p_{hum_{in}}, \dot{m}_{hum_{in}},$
$T_{hum_{in}}, RH_{hum_{in}}$

$p_{hum_{out}}, \dot{m}_{hum_{out}},$
$T_{hum_{out}}, RH_{hum_{out}}$

$P_{air}$

$\dot{m}_{evap}$

$P_{evap}$

Water Tank 64

$P_{w_{loss}}$

$m_w, c_w, T_w$

Water 66

68

$P_{hp}$

$P_{hp_{loss}}$

$C_{hp}, T_{hp}$   Heater Plate

$P_{el}$

70

— Thermal energy transfers
— Mass transfer

FIG. 3

EP 4 547 301 B1

| System parameters | Description | Units |
|---|---|---|
| $A_s(m_w)$ | Water-air interface surface area | $[m^2]$ |
| $\alpha_{hp_{loss}}$ | Heater plate heat loss coefficient | $[W/K]$ |
| $\alpha_{w_{loss}}$ | Water heat loss coefficient | $[W/K]$ |
| $k(Q, m_w)$ | Mass transfer coefficient | $[m/s]$ |
| $h(Q, m_w)$ | Heat transfer coefficient | $[W/(m^2 \cdot K)]$ |

FIG. 4

| Physical constants/functions | Description | Units |
|---|---|---|
| $M_w$ | Molar mass water | [kg/mol] |
| $R_u$ | Universal gas constant | [J/(mol·K)] |
| $R_d$ | Specific gas constant dry air | [J/(kg·K)] |
| $R_v$ | Specific gas constant water vapor | [J/(kg·K)] |
| $L_h$ | Latent heat of evaporation water | [J/kg] |
| $p_{v,s}(T_{air})$ | Saturated vapor pressure | [Pa] |
| $\rho_{air}(p_{air}, T_{air}, RH_{air})$ | Air density | [kg/m³] |

FIG. 5

118

| Variables $_{120}$ | Description $_{122}$ | Units $_{124}$ | Y/N $^{(1)}$ $_{126}$ |
|---|---|---|---|
| $V_{hp}$ | Heater plate coil voltage | [V] | Y |
| $PWM\%$ | PWM duty cycle of heater plate coil | [%] | Y |
| $R_{hp}$ | Heater plate coil electrical resistance | [Ω] | Y |
| $T_{hp}$ | Heater plate temperature | [K] | Y |
| $p_{atm}$ | Ambient pressure | [Pa] | Y |
| $T_{atm}$ | Ambient temperature | [K] | Y |
| $RH_{atm}$ | Ambient relative humidity | [%] | Y |
| $Q$ | Average flow rate | [m³/s] | Y |
| $m_w$ | Water mass | [kg] | N** |
| $\dot{m}_w$ | Evaporation rate | [kg/s] | N |
| $T_w$ | Water temperature | [K] | N |
| $T_{hum_{in}}$ | Humidifier inlet temperature | [K] | Y*** |
| $T_{hum_{out}}$ | Humidifier outlet temperature | [K] | N |

Note (1):
Predictable/measurable
during operation [Y/N].

FIG. 6

FIG. 7

FIG. 8

FIG. 9

| DoE | Run | Target settings | | | | | Known variables during operation | | | | | | | Unknown variables during operation, i.e. to be predicted | | | |
| | | Flow rate setting [L/min] | Heater plate power setting [W] | Pressure setting [cmH2O] | Ambient temperature setting [degC] | Ambient relative humidity setting [%] | Flow rate [L/min] | Heater plate input power [W] | Pressure [cmH2O] | Ambient temperature [degC] | Ambient relative humidity [%] | Ambient pressure [mbar] | Heater plate temperature [degC] | Evaporation rate [mg/s] | Water temperature [degC] | Humidifier inlet temperature [degC] | Humidifier outlet temperature [degC] |
|---|---|---|---|---|---|---|---|---|---|---|---|---|---|---|---|---|---|
| | 1 | 20 | 20 | 4 | - | - | 26.5 | 18.4 | 4.2 | 21.8 | 38.0 | 1013.0 | 47.1 | -5.0 | 36.4 | 26.0 | 28.7 |
| | 2 | 20 | 40 | 4 | - | - | 18.6 | 40.1 | 4.1 | 22.6 | 42.4 | 1022.7 | 71.0 | -10.2 | 54.8 | 29.2 | 37.9 |
| | 3 | 60 | 20 | 4 | - | - | 59.3 | 19.8 | 5.3 | 20.9 | 47.2 | 1021.7 | 43.1 | -6.0 | 30.6 | 24.0 | 25.4 |
| | 4 | 60 | 40 | 4 | - | - | 62.4 | 38.9 | 5.5 | 20.9 | 40.1 | 1027.1 | 59.3 | -11.2 | 40.3 | 24.1 | 27.6 |
| 1 | 5 | 20 | 20 | 20 | - | - | 22.8 | 20.0 | 20.2 | 21.7 | 48.1 | 1022.5 | 50.6 | -5.6 | 40.6 | 34.6 | 34.7 |
| | 6 | 20 | 40 | 20 | - | - | 20.1 | 37.7 | 20.2 | 22.8 | 36.7 | 1026.3 | 68.4 | -10.8 | 54.0 | 37.7 | 40.7 |
| | 7 | 60 | 20 | 20 | - | - | 63.2 | 19.6 | 21.5 | 22.5 | 37.4 | 1015.2 | 42.9 | -6.8 | 31.4 | 30.2 | 29.5 |
| | 8 | 60 | 40 | 20 | - | - | 68.0 | 38.3 | 21.7 | 21.2 | 37.3 | 1025.7 | 57.8 | -12.0 | 40.2 | 29.4 | 31.1 |
| | 9 | 40 | 30 | 12 | - | - | 40.2 | 29.0 | 12.6 | 21.2 | 35.5 | 1022.9 | 54.1 | -7.8 | 39.9 | 27.6 | 30.3 |

FIG. 10

| System parameters | Description | Value | Units |
|---|---|---|---|
| $A_s$ | Water-air interface surface area | 0.0125 | [m2] |
| $\alpha_{hp_{loss}}$ | Heater plate heat loss coefficient | 0.140030 | [W/K] |
| $\alpha_{w_{loss}}$ | Water heat loss coefficient | 0.142785 | [W/K] |
| $k(Q)$ | Mass transfer coefficient | $0.0039397 + 16.161 \cdot Q$ | [m/s] |
| $h(Q)$ | Heat transfer coefficient | $3.7424 + 14872 \cdot Q$ | [W/(m2*K)] |
| $V_{hp}$ | Heater plate coil voltage | 11.7 | [V] |
| $R_{hp}(T_{hp})$ | Heater plate coil resistance | $3.0778 + 0.0021 \cdot (T_{hp} - 273.15)$ | [Ω] |
| $f(\Delta p, Q)$ | Heat transfer from blower to air | $1974.95 \cdot Q + 0.0020533 \cdot \Delta p + 1.6391 \cdot Q \cdot \Delta p$ | [W] |

180

182 184 186 188

FIG. 11

EP 4 547 301 B1

| | | | | | | | | Measured values | | | | Predicted values | | | |
|---|---|---|---|---|---|---|---|---|---|---|---|---|---|---|---|
| Run | Flow rate [l/min] | Heater plate power [W] | Pressure [cmH2O] | Ambient temperature [degC] | Ambient Pressure [mbar] | Ambient relative humidity [%] | Heater plate Temperature [degC] | Evaporation rate [mg/s] | Water temperature [degC] | Humidifier inlet temperature [degC] | Humidifier outlet temperature [degC] | Evaporation rate [mg/s] | Water temperature [degC] | Humidifier inlet temperature [degC] | Humidifier outlet temperature [degC] |
| 11 | 46.0 | 30.8 | 12.8 | 31.3 | 37.6 | 1005.8 | 57.7 | -10.2 | 42.3 | 36.6 | 36.9 | -10.0 | 43.6 | 37.7 | 39.0 |
| 12 | 47.1 | 30.5 | 12.8 | 10.9 | 67.3 | 1006.8 | 51.1 | -7.2 | 35.3 | 17.0 | 21.1 | -7.2 | 36.0 | 16.8 | 20.6 |
| 13 | 43.7 | 30.6 | 12.7 | 30.7 | 80.9 | 1005.4 | 60.1 | -9.2 | 46.1 | 36.5 | 38.0 | -9.2 | 47.1 | 37.3 | 39.4 |

**FIG. 12**

| | | | | | | | | Measured values | | | | Predicted values | | | |
|---|---|---|---|---|---|---|---|---|---|---|---|---|---|---|---|
| Run | Flow rate [l/min] | Heater plate power [W] | Pressure [cmH2O] | Ambient temperature [degC] | Ambient Pressure [mbar] | Ambient relative humidity [%] | Heater plate Temperature [degC] | Evaporation rate [mg/s] | Water temperature [degC] | Humidifier inlet temperature [degC] | Humidifier outlet temperature [degC] | Evaporation rate [mg/s] | Water temperature [degC] | Humidifier inlet temperature [degC] | Humidifier outlet temperature [degC] |
| 14 | 41.9 | 30.6 | 12.7 | 21.8 | 1024.0 | 42.5 | 52.6 | -8.6 | 40.7 | 27.7 | 30.2 | -8.7 | 41.2 | 28.1 | 30.9 |
| 15 | 40.1 | 30.6 | 12.6 | 22.1 | 1020.3 | 42.3 | 57.5 | -8.2 | 41.0 | 27.9 | 30.4 | -8.5 | 41.3 | 28.6 | 31.3 |
| 16 | 40.7 | 30.8 | 12.7 | 21.9 | 1020.2 | 42.7 | 65.0 | -8.0 | 39.9 | 27.9 | 30.2 | -8.2 | 40.5 | 28.4 | 30.9 |

**FIG. 13**

FIG. 14

210

| Description | Unit | 0-3600 | 3600-7200 | 7200-10800 | 10800-14400 | 14400-18000 | 18000-21600 | 21600-25200 | 25200-28800 | |
|---|---|---|---|---|---|---|---|---|---|---|
| Avg. water mass | [g] | N/A | 252.9 | 220.0 | 187 | 154.6 | 121.6 | 89 | 56.4 | N/A |
| Avg. surface water reservoir | [m2] | N/A | 0.0153 | 0.0148 | 0.0143 | 0.0138 | 0.0131 | 0.0124 | 0.0116 | -25% |
| Avg. heater plate temperature | [degC] | N/A | 51.3 | 52.0 | 53.1 | 54.1 | 54.9 | 54.9 | 55.2 | +8% |
| Avg. water temperature | [degC] | N/A | 32.5 | 32.6 | 34.5 | 35.4 | 35.6 | 37.5 | NaN* | +15% |
| Avg. humidifier inlet temperature | [degC] | N/A | 23.3 | 23.1 | 22.9 | 23.0 | 23.0 | 23.1 | 23.1 | ~constant |
| Avg. humidifier outlet temperature | [degC] | N/A | 25.1 | 24.7 | 24.5 | 24.6 | 24.5 | 24.7 | 24.8 | ~constant |
| Avg. evaporation rate | [mg/s] | N/A | 9.07 | 9.25 | 9.06 | 9.17 | 9.09 | 9.06 | 9.12 | ~constant |

FIG. 15

EP 4 547 301 B1

| Variables | Unit | Start | Stop | # of steps |
|-----------|------|-------|------|-----------|
| $PWM\%$ | [%] | 10 | 100 | 5 |
| $Q$ | [L/min] | 20 | 60 | 5 |
| $\Delta p$ | [cmH2O] | 5 | 20 | 5 |
| $p_{atm}$ | [mbar] | 950 | 1050 | 5 |
| $T_{atm}$ | [degC] | 15 | 35 | 5 |
| $RH_{atm}$ | [%] | 10 | 90 | 5 |
| $T_{hp}$ | [degC] | 40 | 70 | 5 |

FIG. 16

FIG. 17

FIG. 18

FIG. 19

FIG. 20

FIG. 21

FIG. 22

FIG. 23

FIG. 24

FIG. 25

**EP 4 547 301 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200338298 A1
- WO 2021135942 A1

- US 2019217044 A1